(19) Europäisches Patentamt
European Patent Office
Office européen des brevets

(11) Veröffentlichungsnummer: **0 275 866**
**A1**

(12) **EUROPÄISCHE PATENTANMELDUNG**

(21) Anmeldenummer: **88100037.6**

(22) Anmeldetag: **05.01.88**

(51) Int. Cl.⁴: **C07D 249/08** , C07D 233/56 , A01N 43/653 , A01N 43/50 , C07F 7/18

(30) Priorität: **14.01.87 DE 3700916**

(43) Veröffentlichungstag der Anmeldung: **27.07.88 Patentblatt 88/30**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Kraatz, Udo, Dr.**
**Andreasstrasse 22a**
**D-5090 Leverkusen(DE)**
Erfinder: **Behrenz, Wolfgang, Dr.**
**Untergruendemich 14**
**D-5063 Overath(DE)**
Erfinder: **Fedtke, Carl, Dr.**
**Werheiderstrasse 5**
**D-5000 Köln 80(DE)**

(54) **Halogenalkyl-, Alkenyl- und Alkinyl-azole.**

(57) Die vorliegende Erfindung betrifft neue Halogenalkyl-, Alkenyl-und Alkinyl-azole der allgemeinen Formel (I)

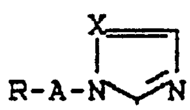

(I)

in welcher
X für ein Stickstoffatom oder eine CH-Gruppierung steht,
R für gegebenenfalls substituiertes tert.-Alkyl oder für gegebenenfalls substituiertes Cycloalkyl steht und
A für eine der Gruppierungen

-C≡C-, 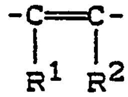

oder

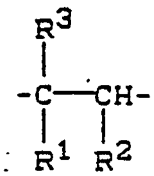 steht,

EP 0 275 866 A1

steht,
worin
$R^1$ für Halogen, Alkoxy oder Trialkylsilyloxy steht,
$R^2$ für Wasserstoff oder Halogen steht und
$R^3$ für Halogen steht,
und ihre Salze, welche als Synergisten in Schädlingsbekämpfungsmitteln verwendet werden können.

## Halogenalkyl-, Alkenyl-und Alkinyl-azole

Die vorliegende Erfindung betrifft neue Halogenalkyl-, Alkenyl-und Alkinyl-azole, mehrere Verfahren zu ihrer Herstellung und ihre Verwendung in Mitteln zur Bekämpfung von Schädlingen.

Es ist bereits bekannt geworden, daß bestimmte Herbizide, wie z. B. 4-Amino-6-tert.-butyl-3-methylthio-bzw. -ethylthio-1,2,4-triazin-5-on; 1-Amino-3-(2,2-dimethylpropyl)-6-(ethylthio)-1,3,5-triazin-2,4-dion; 6-Chlor-2-ethylamino-4-isopropylamino-1,3,5-triazin; 1-Methoxy-1-methyl-3-(3,4-dichlorphenyl)-harnstoff; 1,3-Dimethyl-1-(benzo-1,3-thiazol-2-yl)-harnstoff oder 3-Cyclohexyl-5,6-trimethylenuracil, photosynthese-hemmende Eigenschaften besitzen (vgl. z. B. Carl Fedtke, Biochemistry and Physiology of Herbicide Action, Springer Verlag, 1982). Nachteilig bei diesen herbiziden Verbindungen ist jedoch, daß nicht immer alle vorkommenden Unkräuter und Ungräser voll erfaßt werden oder aber, daß bei entsprechend hohen Aufwandmengen einige Kulturpfanzenarten teilweise geschädigt werden.

Synergistische Mischungen von insektiziden Wirkstoffen, z. B. von Pyrethroiden, mit bestimmten Methylendioxyphenyl-Derivaten, z. B. Piperonylbutoxid, als Synergisten sind ebenfalls bereits bekannt geworden (vgl. z. B. K. Naumann, Chemie der Pflanzenschutz-und Schädlingsbekämpfungsmittel, Springer-Verlag Berlin, Band 7 (1981) Seiten 3 - 6). Die Wirksamkeit dieser Mittel ist jedoch unter den in der Praxis auftretenden Bedingungen nicht immer voll befriedigend.

Es wurden nun neue Halogenalkyl-, Alkenyl-und Alkinylazole der allgemeinen Formel (I)

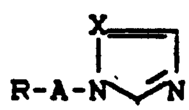

(I)

in welcher
X für ein Stickstoffatom oder eine CH-Gruppierung steht,
R für gegebenenfalls substituiertes tert.-Alkyl oder für gegebenenfalls substituiertes Cycloalkyl steht und
A für eine der Gruppierungen $-C \equiv C-$,

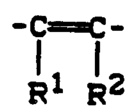

oder

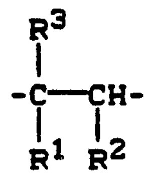

steht,
worin
$R^1$ für Halogen, Alkoxy oder Trialkylsilyloxy steht,
$R^2$ für Wasserstoff oder Halogen steht und
$R^3$ für Halogen steht,
und ihre Salze gefunden.

Unter Salzen der Verbindungen der Formel (I) sollen im folgenden die Säureadditions-Salze und Metallsalz-Komplexe verstanden werden.

Weiterhin wurde gefunden, daß man die Halogenalkyl-, Alkenyl-und Alkinyl-azole der Formel (I)

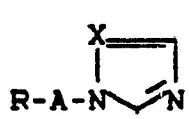

(I)

in welcher
X, R und A die oben angegebenen Bedeutungen haben,
und ihre Salze erhält, wenn man
(a) für den Fall, daß X und R die oben angegebenen Bedeutungen haben und
A für die Gruppierung

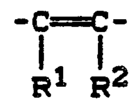

steht, worin
$R^1$ für Halogen steht und
$R^2$ für Wasserstoff steht,
Azolyl-Ketone der allgemeinen Formel (II)

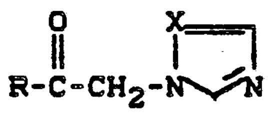
(II)

in welcher
X und R die oben angegebenen Bedeutungen haben,
in an sich bekannter Weise mit geeigneten Halogenierungsmitteln umsetzt, oder
(b) für den Fall, daß X und R die oben angegebenen Bedeutungen haben und
A für die Gruppierung

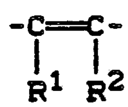

steht, worin
$R^1$ für Alkoxy oder Trialkylsilyloxy steht und
$R^2$ für Wasserstoff steht,
Azolyl-Ketone der allgemeinen Formel (II)

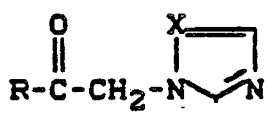
(II)

in welcher
X und R die oben angegebenen Bedeutungen haben,
in an sich bekannter Weise in Gegenwart einer starken Base und in Gegenwart eines Verdünnungsmittels mit einem Alkylierungsmittel bzw. einem Silylierungsmittel umsetzt, oder
(c) für den Fall, daß X und R die oben angegebenen Bedeutungen haben und
A für die Gruppierung -C≡C-steht,
Chlorvinyl-azol-Derivate der allgemeinen Formel (Ia)

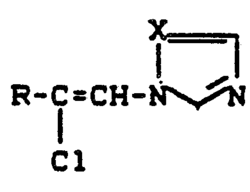
(Ia)

in welcher
X und R die oben angegebenen Bedeutungen haben,
durch Umsetzung mit einer starken Base in Gegenwart eines Verdünnungsmittels dehydrochloriert, oder

(d) für den Fall, daß X und R die oben angegebenen Bedeutungen haben und
A für die Gruppierung

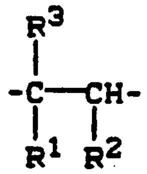

steht, worin
$R^1$, $R^2$ und $R^3$ die oben angegebene Bedeutung haben,
Alkenyl-azole der allgemeinen Formel (Ib)

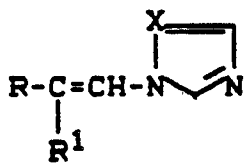

(Ib)

in welcher
X, R und $R^1$ die oben angegebenen Bedeutungen haben,
mit Halogenverbindungen der Formel (III)
$R^2$-$R^3$ (III)
in welcher
$R^2$ und $R^3$ die oben angegebene Bedeutung haben,
in Gegenwart eines Verdünnungsmittels umsetzt, oder
(e) für den Fall, daß X und R die oben angegebenen Bedeutungen haben und
A für die Gruppierung

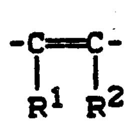

steht, worin
$R^1$ die oben angegebene Bedeutung hat und
$R^2$ für Halogen steht,
Halogenalkyl-azole der Formel (Ic)

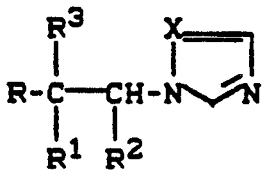

(Ic)

in welcher
X, R und $R^1$ die oben angegebenen Bedeutungen haben und
$R^2$ und $R^3$ für Halogen stehen,
durch Umsetzung mit einer starken Base in Gegenwart eines Verdünnungsmittels dehydrohalogeniert und gegebenenfalls an die so erhaltenen Verbindungen der Formel (I) eine Säure oder ein Metallsalz nach üblichen Methoden addiert.

Die vorliegende Erfindung betrifft weiterhin die Verwen dung von Verbindungen der allgemeinen Formel (I) und ihren Salzen als Synergisten zur Bekämpfung von Schädlingen, vorzugsweise von pflanzlichen und besonders bevorzugt von tierischen Schädlingen, sowie synergistische Mischungen aus Verbindungen der Formel (I) und ihren Salzen und bekannten Photosynthesehemmer-Herbiziden (zur Unkrautbekämpfung) und besonders bevorzugt synergistische Mischungen aus Verbindungen der Formel (I) und ihren Salzen und bekannten Arthropodiziden, insbesondere Insektiziden und Akariziden (zur Bekämpfung von Arthropoden) und die Verwendung dieser Mischungen zur Schädlingsbekämpfung, vorzugsweise zur Bekämpfung von Arthropoden.

Gegenstand der vorliegenden Erfindung sind auch die neuen herbiziden Mittel, welche bekannte Photosynthesehemmer-Herbizide einerseits und erfindungsgemäße Verbindungen der Formel (I) und ihre Salze als Synergisten andererseits enthalten sowie die Verwendung dieser Mittel als Herbizide.

Weiterhin betrifft die vorliegende Erfindung besonders bevorzugt neue Schädlingsbekämpfungsmittel, welche die Verbindungen der Formel (I) und ihre Salze als Synergisten neben arthropodiziden, insbesondere insektiziden und akariziden Wirkstoffen enthalten und die Verwendung dieser Mittel zur Schädlingsbekämpfung.

Als Arthropodizide (gegen Arthropoden wirksame Stoffe) kommen praktisch alle üblichen Wirkstoffe in Frage (vgl. z. B. K.-H. Büchel, Pflanzenschutz und Schädlingsbekämp fungsmittel, Thieme Verlag Stuttgart, 1977, und Farm Chemicals Handbook 1979, Meister Publishing Co, Willoughby, 1979).

Bevorzugt werden die Verbindungen der allgemeinen Formel (I) und ihre Salze zusammen mit arthropodiziden

1. Carbamidsäureestern und/oder
2. Carbonsäureestern einschließlich der natürlichen sowie synthetischen Pyrethroide und/oder
3. Phosphorverbindungen, wie Phosphorsäure-und Phosphonsäureestern einschließlich der Thiol-und Thiono-Verbindungen verwendet.

Die neuen Verbindungen der allgemeinen Formel (I) und ihre Salze besitzen bei den üblichen Aufwandmengen zwar keine eigene herbizide Wirkung, bewirken aber - wie ebenfalls gefunden wurde - eine wesentliche Steigerung der herbiziden Wirkung von Photosynthesehemmer-Wirkstoffen. Der hier aufgefundene synergistische Effekt ist völlig unerwartet und überraschend.

Da der neu aufgefundene synergistische Effekt auch solche Unkräuter betrifft, die durch die verwendeten Photosynthesehemmer-Wirkstoffe bei alleiniger Ausbringung in üblichen Aufwandmengen nur unzureichend geschädigt oder gar nicht erfaßt werden, stellen die erfindungsgemäßen synergistischen Wirkstoffkombinationen eine wertvolle Bereicherung der Technik dar.

Überraschenderweise ist auch die Wirkung der erfindungsgemäßen Wirkstoffkombinationen aus Verbindungen der Formel (I) und ihren Salze und bekannten Arthropodiziden gegen Arthropoden wesentlich höher als die Wirkung dieser Einzelkomponenten bzw. die Summe der Wirkungen der Einzelkomponenten. Sie ist ferner wesentlich höher als die Wirkung von Wirkstoffkombinationen mit dem bekannten handelsüblichen Synergisten Piperonylbutoxid. Die erfindungsgemäß verwendbaren Verbindungen der Formel (I) und ihre Salze zeigen ausgezeichnete synergistische Wirksamkeit nicht nur bei einer Wirkstoffklasse, sondern bei Wirkstoffen aus den verschiedensten chemischen Stoffgruppen.

Die synergistische Wirkung der Verbindungen der allgemeinen Formel (I) und ihrer Salze zeigt sich besonders bevorzugt bei

1) Carbamidsäureestern der Formel (IV)

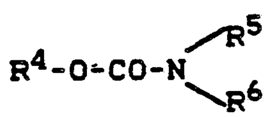

(IV)

in welcher

$R^4$ für einen gegebenenfalls substituierten carbocyclischen oder heterocyclischen aromatischen Rest oder für einen gegebenenfalls substitu ierten Oximrest steht (wobei die weiter unten erläuterten Rest $R^4$ bevorzugt werden),

$R^5$ für $C_1$-$C_4$-Alkyl steht und

$R^6$ für Wasserstoff, $C_1$-$C_4$-Alkyl oder für einen Rest Y steht, wobei

Y für den Rest -CO-$R^7$ steht, worin

$R^7$ für Halogen, $C_1$-$C_4$-Alkyl, $C_1$-$C_4$-Alkoxy, $C_3$-$C_5$-Alkenoxy, $C_3$-$C_5$-Alkinoxy, $C_1$-$C_4$-Alkylthio, $C_1$-$C_4$-Alkylamino, Di-$C_1$-$C_4$-Alkylamino, $C_1$-$C_4$-Alkyl-hydroxylamino, für gegebenenfalls durch Halogen, Nitro, Cyano, Trifluoromethyl, $C_1$-$C_4$-Alkyl, $C_1$-$C_4$-Alkoxy, $C_1$-$C_4$-Alkylendioxy, $C_1$-$C_4$-Alkylthio, $C_1$-$C_4$-Alkoxy-carbonyl substituiertes Phenoxy, Phenylthio oder Phenylamino, für 2,3-Dihydro-2,2-dimethyl-7-benzofuranyl oder für den Rest

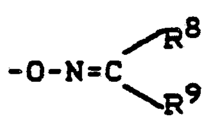

steht, worin
$R^8$ für Wasserstoff, $C_1$-$C_4$-Alkyl oder Di-$C_1$-$C_4$-alkylamino-carbonyl steht und
$R^9$ für $C_1$-$C_4$-Alkyl, $C_1$-$C_4$-Alkyl thio, Cyano-$C_1$-$C_4$-alkylthio, $C_1$-$C_4$-Alkylthio-$C_1$-$C_4$-alkyl steht,
oder die beiden Reste $R^8$ und $R^9$ zusammen für gegebenenfalls durch Sauerstoff, Schwefel, SO oder $SO_2$ unterbrochenes $C_2$-$C_8$-Alkandiyl stehen, oder
in welcher
Y für den Rest $-S_o(O)_p-R^{10}$ steht, worin
o für 1 oder 2 und
p für 0, 1 oder 2 stehen (wenn o für 2 steht, bedeutet p 0) und
$R^{10}$ für gegebenenfalls durch Halogen substituiertes $C_1$-$C_4$-Alkyl, $C_3$-$C_5$-Alkenyl, $C_3$-$C_5$-Alkinyl oder $C_3$-$C_6$-Cycloalkyl, für gegebenenfalls durch Halogen, Cyano, Nitro, Trifluormethyl, Trifluormethoxy, Trifluormethylthio, $C_1$-$C_4$-Alkyl oder $C_1$-$C_4$-Alkoxy substituiertes Phenyl, Benzyl oder Phenylethyl oder für den Rest

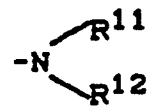

steht, worin
$R^{11}$ für $C_1$-$C_4$-Alkyl, $C_3$-$C_5$-Alkenyl, $C_3$-$C_5$-Alkinyl, $C_3$-$C_6$-Cycloalkyl oder Benzyl steht und
$R^{12}$ für $C_1$-$C_4$-Alkyl, $C_3$-$C_5$-Alkenyl, $C_3$-$C_5$-Alkinyl, $C_3$-$C_6$-Cycloalkyl, Benzyl, Phenylethyl, Halogencarbonyl, Formyl, $C_1$-$C_4$-Alkyl-carbonyl, $C_1$-$C_4$-Alkoxy-carbonyl, $C_1$-$C_4$-Alkoxyphenoxy-carbonyl, $C_3$-$C_5$-Alkinoxy-carbonyl, $C_3$-$C_5$-Alkenoxy-carbonyl, $C_1$-$C_4$-Alkylthiocarbonyl, $C_1$-$C_4$-Alkylamino-carbonyl, $C_1$-$C_4$-Alkyl-hydroxylamino-carbonyl, $C_1$-$C_{10}$-Alkyl-phenoxycarbonyl, Di-$C_1$-$C_4$-alkyl-amino-carbonyl, Phenylthio-carbonyl, Phenoxy-carbonyl, 2,3-Dihydro-2,2-dimethyl-7-benzofuranyloxy-carbonyl, für gegebenenfalls durch Halogen, Cyano, Nitro, Trifluormethyl, $C_1$-$C_{10}$-Alkyl oder $C_1$-$C_4$-Alkoxy substituiertes Phenylsulfenyl, Phenylsulfinyl, Phenylsulfonyl oder Phenyl steht, oder für den Rest

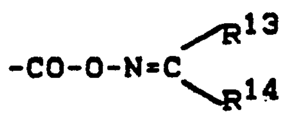

steht, worin
$R^{13}$ die oben für $R^8$ angegebene Bedeutung und
$R^{14}$ die oben für $R^9$ angegebene Bedeutung hat,
wobei ferner im Rest

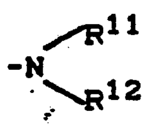

die Reste $R^{11}$ und $R^{12}$ zusammen für eine gegebenenfalls durch Sauerstoff oder Schwefel unterbrochene Kohlenwasserstoffkette mit 3 bis 8 Kohlenstoffatomen stehen und worin weiter $R^{10}$ auch für den gleichen Rest stehen kann, an den der Rest $-S_o(O)_p-R^{10}$ gebunden ist.

Als Wirkstoffkomponenten ganz besonders bevorzugt sind Carbamidsäureester der Formel (IV), in welcher
$R^4$ für gegebenenfalls durch $C_1$-$C_4$-Alkyl, $C_2$-$C_4$-Alkenyl, $C_1$-$C_4$-Alkoxy, $C_1$-$C_4$-Alkoxy-methyl, $C_1$-$C_4$-Alkylthio, $C_1$-$C_4$-Alkylthio-methyl, $C_1$-$C_4$-Alkylamino, Di-($C_1$-$C_4$-alkyl)-amino, Di-($C_3$-$C_4$-alkenyl)-amino, Halogen, Dioxolanyl, Methylendioxy und/oder durch den Rest $-N=CH-N(CH_3)_2$ substituierte Rest aus der Reihe Phenyl, Naphthyl, 2,3-Dihydro-7-benzofuranyl, Pyrazolyl oder Pyrimidinyl steht, oder in welcher $R^4$ für einen Alkylidenaminorest der Formel

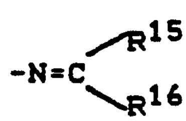

steht,

in welcher
$R^{15}$ und $R^{16}$ die oben für $R^8$ bzw. $R^9$ angegebene Bedeutung haben, und
$R^5$ für $C_{1-4}$-Alkyl steht und
$R^6$ für Wasserstoff oder $C_1$-$C_4$-Alkyl (vorzugsweise für Wasserstoff) steht.

Als Beispiele für die Carbamidsäureester der Formel (IV) seien die folgenden N-Methylcarbamidsäureester genannt:
2-Methyl-phenyl-, 2-Ethyl-phenyl-, 2-iso-Propyl-phenyl-, 2-sec-Butyl-phenyl-, 2-Methoxy-phenyl-, 2-Ethoxy-phenyl-, 2-iso-Propoxy-phenyl-, 4-Methyl-phenyl-, 4-Ethyl-phenyl-, 4-n-Propyl-phenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylaminophenyl-, 2-Ethylthiomethyl-phenyl-, 1-Naphthyl-, 2,3-Dihydro-2,2-dimethyl-7-benzofuranyl, 2,3-(Dimethyl-methylendioxy)-phenyl-, 2-(4,5-Dimethyl-1,3-dioxolan-2-yl)-phenyl-, 1-Methylthioethyliden-amino-, 2-Methylthio-2-methylpropylidenamino-, 1-(2-Cyanoethylthio)-ethylidenamino-und 1-Methylthiomethyl-2,2-dimethyl-propylidenamino-N-methyl-carbamidsäureester.

Die synergistische Wirkung der Verbindungen der allgemeinen Formel (I) zeigt sich weiter bevorzugt bei
2) Carbonsäureestern der Formel (V)

$$R^{17}\text{-CO-O-}\overset{R^{18}}{\overset{|}{C}}H - R^{19} \qquad (V)$$

in welcher
$R^{17}$ für einen offenkettigen oder cyclischen Alkylrest steht, der gegebenenfalls substituiert ist durch Halogen, Alkyl, Cycloalkyl, durch gegebenenfalls durch Halogen, Alkyl und/oder Alkoxy substituiertes Alkenyl, durch Phenyl oder Styryl, welche gegebenenfalls durch Halogen, gegebenenfalls Halogen-substituierte Reste aus der Reihe Alkyl, Alkoxy, Alkyldendioxy und/oder Alkylthio substituiert sind, durch spirocyclisch verknüpftes, gegebenenfalls Halogen-substituiertes Cycloalk(en)yl, welches gegebenenfalls benzannelliert ist, in welcher weiter
$R^{18}$ für Wasserstoff, Alkyl, Halogenalkyl, Alkenyl, Alkinyl oder Cyano steht, und
$R^{19}$ für einen gegebenenfalls substituierten Alkyl-oder Arylrest oder für einen Heterocyclus steht, oder zusammen mit $R^{18}$ und dem Kohlenstoffatom, an das beide Reste gebunden sind, einen Cyclopentenonring bildet.

Ganz besonders als Wirkstoffkomponenten bevorzugt sind Carbonsäureester der Formel (V), in welcher
$R^{17}$ (a) für den Rest

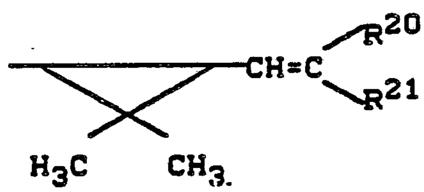

steht, worin
$R^{20}$ für Wasserstoff, Methyl, Fluor, Chlor und Brom steht und
$R^{21}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls Halogen-substituierte Reste der Reihe $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht, oder worin beiden Reste $R^{20}$ und $R^{21}$ für $C_2$-$C_5$-Alkandiyl (Alkylen) stehen;
oder in welcher
$R^{17}$ (b) für den Rest - $\underset{R^{23}}{\underset{|}{C}}H - R^{22}$ steht, worin
$R^{22}$ für gegebenenfalls durch Halogen und/oder durch gegebenenfalls Halogen-substituierte Reste der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und
$R^{23}$ für Isopropyl oder Cyclopropyl steht;
oder in welcher
$R^{17}$ (c) für Methyl oder einen der Reste

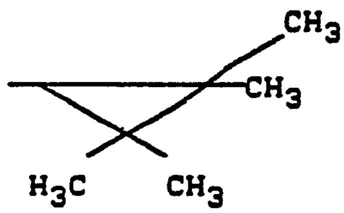 , 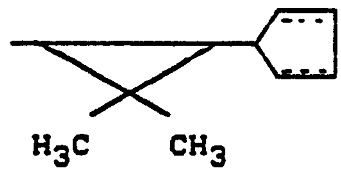 ,

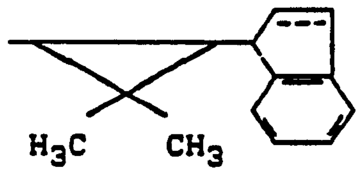

wobei die gepunkteten Linien mögliche Doppelbindungen andeuten sollen, steht,
und in welcher
$R^{18}$ für Wasserstoff, $C_1$-$C_4$-Alkyl, $C_1$-$C_4$-Halogenal kyl Cyano oder Ethinyl steht und
$R^{19}$ für die Reste der Reihe Phenyl, Furyl oder Tetrahydrophthalimido steht, wobei diese Reste substituiert sein können durch Halogen und/oder Reste der Reihe $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl, welche ihrerseits durch Halogen substituiert sein können und wobei $R^{19}$ vorzugsweise für 2,3,5,6-Tetrafluorphenyl oder 4-Methylthio-2,3,5,6-tetrafluorphenyl, 3,4-Dichlorphenyl, Tetrahydrophthalimido oder für Phenoxyphenyl, steht, welches in einem oder beiden Phenylringen durch Halogen substituiert sein kann.

Weiter sind die natürlich vorkommenden Pyrethroide (wie Pyrethreum) als Carbonsäureester der Formel (V) besonders bevorzugt.

Als Beispiele für die Carbonsäureester der Formel (V) seien genannt:
Essigsäure-(2,2,2-trichlor-1-(3,4-dichlor-phenyl)-ethyl)-ester, 2,2-Dimethyl-3-(2-methyl-propen-1-yl)-cyclopropan-carbonsäure-(3,4,5,6-tetrahydrophthalimido-methyl)-ester, 2,2-Dimethyl-3-(2,2-dichlor-vinyl)-cyclopropan-carbonsäure-(3-phenoxybenzyl)-ester, 2,2-Dimethyl-3-(2,2-dichlorvinyl)-cyclopropan-carbonsäure-(α-cyano-3-phenoxy-benzyl)-ester, 2,2-Dimethyl-3-(2,2-dichlorvinyl)-cyclopro pancarbonsäure-(α-cyano-4-fluor-3-phenoxy-benzyl)-ester, 2,2-Dimethyl-3-(2,2-dichlorvinyl)-cyclopropancarbonsäure-(2,3,5,6-tetrafluorbenzyl)-ester, 2-2-Dimethyl-3-(2,2-dibromvinyl)-cyclopropancarbonsäure-(α-cyano-3-phenoxy-benzyl)-ester und 3-Methyl-2-(4-chlor-phenyl)-butan-säure-(α-cyano-3-phenoxy-benzyl)-ester.

Weiter zeigt sich die synergistische Wirkung der Verbindungen der allgemeinen Formel (I) bevorzugt bei
3) Phosphorsäure-und Phosphonsäureestern der allgemeinen Formel (VI)

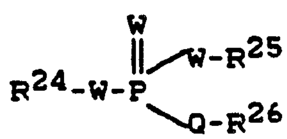

(VI)

in welcher
Q für O, S, -NH-oder für eine direkte Bindung zwischen dem zentralen P-Atom und $R^{26}$ steht, und
$R^{24}$ und $R^{25}$ gleich oder verschieden sind und für gegebenenfalls substituiertes Alkyl oder Aryl stehen, und
$R^{26}$ für Wasserstoff, gegebenenfalls substituiertes Alkyl, Aryl, Heteroaryl, Aralkyl, Alkenyl, Dioxanyl oder einen Oximrest oder für den gleichen Rest steht, an den es gebunden ist, und
W gleich oder verschieden ist und für O oder S steht.

Besonders bevorzugt sind Phosphorsäure-und Phosphonsäureester der Formel (VI), in welcher
$R^{24}$ und $R^{25}$ gleich oder verschieden sind und für $C_1$-$C_4$-Alkyl oder Phenyl stehen,
$R^{26}$ für Wasserstoff oder für Alkyl mit 1 bis 4 Kohlenstoffatomen steht, das gegebenenfalls durch Halogen, Hydroxyl, Cyano, gegebenenfalls Halogen-substituiertes Phenyl, Carbamoyl, Alkylsulfonyl, Alkylsulfinyl, Alkylcarbonyl, Alkoxy, Alkylthio, Alkoxy-carbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen, gegebenenfalls Halogen-substituiertes Phenyl oder $C_1$-$C_4$-Alkoxycarbonyl substituiert ist, oder für den Rest der allgemeinen Formel

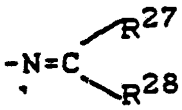

wobei

$R^{27}$ und $R^{28}$ die oben für $R^8$ bzw. $R^9$ angegebene Bedeutung besitzen, oder für Cyano oder Phenyl stehen, und in welcher

$R^{26}$ ferner für Dioxanyl, das durch denselben Rest substituiert ist, an den $R^{25}$ gebunden ist, oder $R^{26}$ für den gleichen Rest an den es gebunden ist steht, oder $R^{26}$ für Phenyl, das gegebenenfalls durch Methyl, Nitro, Cyano, Halogen und/oder Methylthio substituiert ist steht, wobei

$R^{26}$ außerdem besonders bevorzugt für gegebenenfalls durch $C_1$-$C_4$-Alkoxy, $C_1$-$C_4$-Alkylthiomethyl, $C_1$-$C_4$-Alkyl und/oder durch Halogen substituierte heteroaromatische Reste, wie Pyridinyl, Chinolinyl, Chinoxalinyl, Pyrimidinyl oder Benzo-1,2,4-triazinyl steht.

Im einzelnen seien genannt:

O,O-Dimethyl-bzw. OO,O-Diethyl-O-(2,2-dichlor-bzw. 2,2-dibromvinyl)-phosphorsäureester, O,O-Diethyl-O-(4-nitro-phenyl)-thionophosphorsäureester, O,O-Dimethyl-O-(3-methyl-4-methylthio-phenyl)-thionophosphorsäureester, O,O-Dimethyl-O-(3-methyl-4-nitrophenyl)-thionophosphorsäureester, O-Ethyl-S-n-propyl-O-(2,4-dichlorphenyl)-thionophosphorsäureester, O-Ethyl-S-n-propyl-O-(4-methylthio-phenyl)-thionophosphorsäureester, O,O-Dimethyl-S-(4-oxo-1,2,3-benzotriazin(3)yl-methyl)-thionothiolphosphorsäureester, O-Methyl-O-(2-iso-propyl-6-methoxy-pyrimidin(4)yl)-thionomethanphosphonsäureester, O,O-Di ethyl-O-(2-iso-propyl-6-methyl-pyrimidin(4)yl)-thionophosphorsäureester, O,O-Diethyl-O-(3-chlor-4-methyl-cumarin(7)-yl)-thionophosphorsäureester, O,O-Dimethyl-2,2,2-trichlor-1-hydroxy-ethan-phosponsäureester, O,O-Dimethyl-S-(methylaminocarbonylmethyl)-thionophosphorsäureester.

In den allgemeinen Formeln bedeutet gegebenenfalls substituiertes tert.-Alkyl R eine Alkylgruppe mit vorzugsweise 4 bis 12, insbesondere 4 bis 8 Kohlenstoffatomen, wobei wenigstens ein Kohlenstoffatom Bindungen zu 4 direkt benachbarten Kohlenstoffatomen aufweisen muß und wobei diese Alkylgruppe durch einen oder mehrere, vorzugsweise 1 bis 3, insbesondere 1 oder 2 gleiche oder verschiedene Substituenten substituiert sein kann. Als bevorzugte Substituenten seien Halogenatome (Fluor, Chlor, Brom und/oder lod, vorzugsweise Fluor und/oder Chlor) genannt.

Als bevorzgute tert.-Alkylgruppen seien aufgeführt: $(CH_3)_3C$-, Cl-$CH_2$-$C(CH_3)_2$-, F-$CH_2$-$C(CH_3)_2$-, $CH_3$-$C(C_2H_5)_2$- und $(CH_3)_3C$-$CH_2$-$C(CH_3)_2$-.

Gegebenenfalls substituiertes Cycloalkyl R bedeutet Cycloalkyl mit vorzugsweise 3 bis 7, insbesondere 3 bis 6 und ganz besonders bevorzugt 5 oder 6 Ringgliedern (wie Cyclopropyl, Cyclobutyl, Cyclopentyl, Cyclohexyl und Cycloheptyl), wobei der Cycloalkyl-Rest durch einen oder mehrere, vorzugsweise 1 bis 3, insbesondere einen Substituenten substituiert sein kann. Als Substituenten seien Alkyl mit vorzugsweise 1 bis 4, insbesondere 1 oder 2 Kohlenstoffatomen sowie Halogen (Fluor, Chlor, Brom und lod, vorzugsweise Fluor oder Chlor) genannt. Besonders bevorzugt werden 1-Methyl-cyclopentyl und 1-Methyl-cyclohexyl.

In der Definition von A bedeutet Halogen $R^1$, $R^2$ und $R^3$ Fluor, Chlor, Brom und lod, vorzugsweise Fluor, Chlor und Brom, insbesondere Chlor und Brom und besonders bevorzugt Chlor.

Alkoxy $R^1$ bedeutet Alkoxy mit vorzugsweise 1 bis 6, insbesondere 1 bis 4 und besonders bevorzugt 1 oder 2 Kohlenstoffatomen.

Die Alkylgruppen im Trialkylsilyloxy-Rest $R^1$ enthalten vorzugsweise 1 bis 4, insbesondere 1 oder 2 Kohlenstoffatome je Alkylgruppe. Besonders bevorzugt wird der Trimethylsilyloxy-Rest.

A steht besonders bevorzugt für die Gruppen

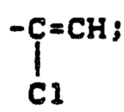 ; 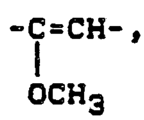 , 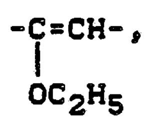 , 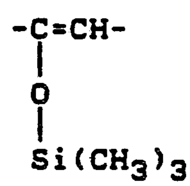 und $-CCl_2-CHCl-$.

X bedeutet vorzugsweise ein Stickstoffatom (welches Bestandteil eines 1,2,4-Triazol-1-yl-Ringes ist).

Als bevorzugte Salze der (basischen) Verbindungen der Formel (I) seien ihre Salze mit Halogenwasserstoffsäuren, wie z. B. die Chlorwasserstoffsäure und Bromwasserstoffsäure, ferner Phosphorsäure, Salpetersäure, mono-und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure sowie Sulfonsäuren, wie z. B. p-Toluolsulfonsäure und 1,5-Naphthalindisulfonsäure, genannt.

Weiterhin seien die Additionsprodukte aus Salzen von Metallen der II. bis IV. Haupt-und der I. und II. sowie IV. bis VIII. Nebengruppe des Periodensystems der Elemente mit den Verbindungen der Formel (I) aufgeführt.

Vorzugsweise liegen die Verbindungen der Formel (I) in freier Form (also nicht als Salz) vor.

In den bevorzugten erfindungsgemäßen Halogenalkyl-, Alkenyl-und Alkinyl-azolen der Formel (I) stehen
X für ein Stickstoffatom oder eine CH-Gruppierung,
R für gegebenenfalls durch Halogen substituiertes tert.-Alkyl mit 4 bis 8 Kohlenstoffatomen oder für gegebenenfalls durch Alkyl mit 1 bis 4 Kohlenstoffatomen und/oder Halogen substituiertes Cycloalkyl mit 3 bis 6 Kohlenstoffatomen und
A für die Gruppierungen

-C≡C-, 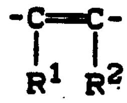 oder 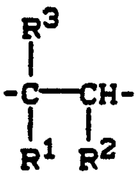

wobei
$R^1$ für Halogen (vorzugsweise Brom oder Chlor), Alkoxy mit 1 bis 4 Kohlenstoffatomen oder für Trimethylsilyloxy steht,
$R^2$ für Wasserstoff oder Halogen (vorzugsweise Brom oder Chlor) steht und
$R^3$ für Halogen (vorzugsweise Brom oder Chlor) steht.

Besonders bevorzugt sind die Verbindungen der Formel (I), in denen
X für ein Stickstoffatom oder eine CH-Gruppierung (vorzugsweise ein Stickstoffatom) steht,
R für gegebenenfalls durch Chlor und/oder Fluor substituiertes tert.-Alkyl mit 4 bis 8 Kohlenstoffatomen (vorzugsweise tert.-Butyl) oder für gegebenenfalls durch Chlor, Methyl, Ethyl und/oder Isopropyl substituiertes Cyclopropyl, Cyclopentyl oder Cyclohexyl steht und
A für die Gruppierung -C≡C-steht oder
A für die Gruppierung

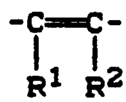

steht,
in welcher
(a) $R^1$ für Chlor, Methoxy, Ethoxy oder Trimethylsilyloxy steht und
$R^2$ für Wasserstoff steht, oder
(b) $R^1$ und $R^2$ für Chlor stehen, oder
A für die Gruppierung

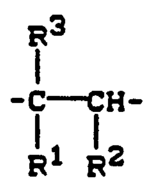

steht,
worin
$R^1$, $R^2$ und $R^3$ für Chlor stehen.

Ganz besonders bevorzugt werden die Verbindungen der Formel (I), in welcher
X für ein Stickstoffatom steht,
R für die Reste $(CH_3)_3C$-, Cl-$CH_2C(CH_3)_2$-, F-$CH_2C(CH_3)_2$-, $CH_3$-$C(C_2H_5)_2$ oder $(CH_3)_3C$-$CH_2$-$C(CH_3)_2$-steht und
A für die Gruppierungen -C≡C-, -CCl = CH-, -$C(OCH_3)$ = CH-, -$C(OC_2H_5)$ = CH-, -$C(OSi(CH_3)_3$ = CH-oder -$CCl_2$-CHCl-steht.

Verwendet man beispielsweise 3,3-Dimethyl-1-(1,2,4-triazol-1-yl)-butan-2-on als Ausgangsstoff sowie

Phosphoroxytrichlorid und Phosphorpentachlorid als Halogenie rungsmittel, so kann der Ablauf der erfindungsgemäßen Verfahrensvariante (a) durch folgendes Formelschema wiedergegeben werden:

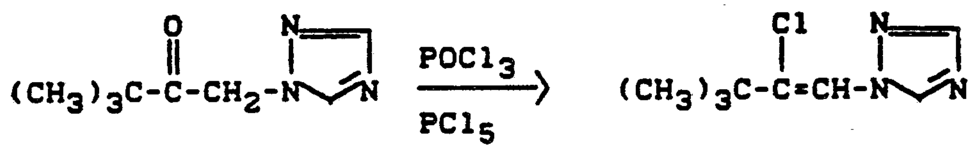


Verwendet man beispielsweise 3,3-Dimethyl-1-(1,2,4-triazol-1-yl)-butan-2-on als Ausgangsstoff, Diethylsulfat als Alkylierungsmittel und Natriumhydrid als starke Base, so kann der Ablauf der erfindungsgemäßen Verfahrensvariante (b) durch folgendes Formelschema wiedergegeben werden:

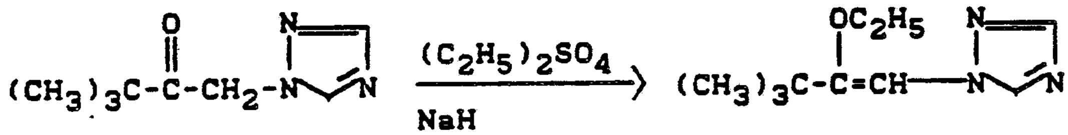


Verwendet man beispielsweise 3,3-Dimethyl-1-(1,2,4-triazol-1-yl)-2-chlorbut-1-en als Ausgangsstoff und Natriumethylat als starke Base, so kann der Ablauf der erfindungsgemäßen Verfahrensvariante (c) durch folgendes Formelschema wiedergegeben werden:

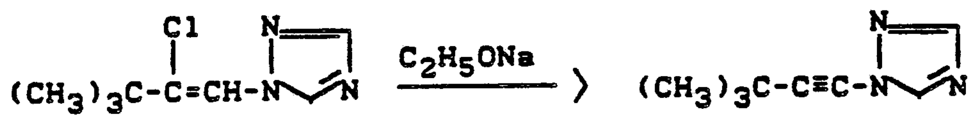


Verwendet man beispielsweise 3,3-Dimethyl-1-(1,2,4-triazol-1-yl)-2-chlor-buten und Brom als Ausgangsstoffe, so kann der Ablauf der erfindungsgemäßen Verfahrensvariante (d) durch folgendes Formelschema wiedergegeben werden:

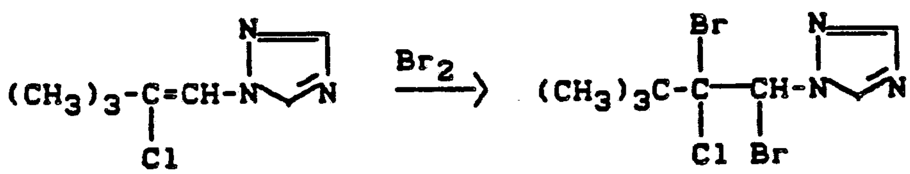


Verwendet man beispielsweise 3,3-Dimethyl-1-(1,2,4-triazol-1-yl)-1,2-dibrom-2-chlor-butan als Ausgangsstoff und Natriumethylat als starke Base, so kann der Ablauf der erfindungsgemäßen Verfahrensvariante (e) durch folgendes Formelschema wiedergegeben werden:

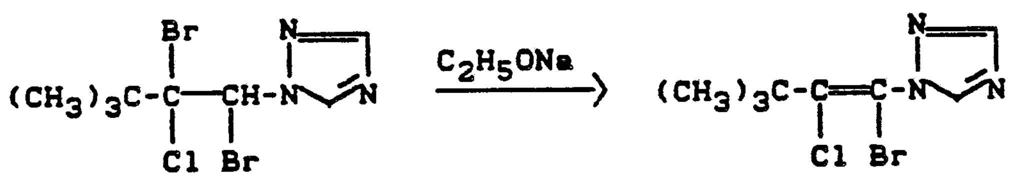


Die für die Durchführung der erfindungsgemäßen Verfahrensvarianten (a) und (b) als Ausgangsstoffe zu verwendenden Azolyl-Ketone sind bekannt (vergl. z. B. DE-OS 2 431 407, EP 0 031 911, EP 0 054 865, DE-OS 2 610 222, DE-OS 2 638 470, DE-OS 2 820 361, DE-OS 3 145 857, DE-OS 3 145 858); bzw. können nach im Prinzip bekannten Verfahren hergestellt werden, wie durch Umsetzung der entsprechenden Halogenmethylketone mit dem entsprechenden Azol in Gegenwart eines inerten organischen Lösungsmittels, wie beispielsweise Aceton oder Acetonitril, und in Gegenwart eines Säurebindemittels, wie beispielsweise Kaliumcarbonat oder Triethylamin, bei Temperaturen zwischen 20 °C und 150 °C.

Die für die Durchführung der erfindungsgemäßen Verfahrensvariante (c) als Ausgangsstoffe zu verwendenden Chlorvinyl-azol-Derivate der Formel (Ia) sind neue Verbindungen und können gemäß den erfindungsgemäßen Verfahrensvarianten (a) und (b) hergestellt werden. Sie sind Bestandteil der vorliegenden Erfindung.

Die für die Durchführung der erfindungsgemäßen Verfahrensvariante (d) als Ausgangsstoffe zu verwendenden Alkenyl-azole der Formel (Ib) sind ebenfalls neue Verbindungen und können gemäß den erfindungsgemäßen Verfahrensvarianten (a) und (b) hergestellt werden. Sie sind ebenfalls Bestandteil der vorliegenden Erfindung.

Die für die Durchführung der erfindungsgemäßen Verfahrensvariante (e) als Ausgangsstoffe zu verwendenden Halogenalkyl-azole der Formel (Ic) sind ebenso neue Verbindungen und können gemäß dem erfindungsgemäßen Verfahren (d) hergestellt werden. Diese Verbindungen sind ebenso Bestandteil der vorliegenden Erfindung.

Als geeignete Halogenierungsmittel für die erfindungsgemäße Verfahrensvariante (a) kommen alle überlicherweise für die Umwandlung von aliphatischen Ketonen in die entsprechenden Vinylhalogenide verwendbaren Halogenierungsmittel, insbesondere Phosphorpentahalogenide, wie beispielsweise Phosphorpentachlorid und Phosphorpentabromid, sowie Phosphoroxytrihalogenide, wie beispielsweise Phosphoroxytrichlorid und Phosphoroxytribromid, in Frage.

Die erfindungsgemäße Verfahrensvariante (a) wird vorzugsweise ohne Verdünnungsmittel durchgeführt.

Die Reaktionstemperaturen können bei der Durchführung der erfindungsgemäßen Verfahrensvariante (a) in größerem Bereich variiert werden. Im allgemeinen arbeitet man bei Temperaturen zwischen 20 °C und 200 °C, vorzugsweise bei Temperaturen zwischen 40 °C und 180 °C.

Bei der Durchführung der erfindungsgemäßen Verfahrensvariante (a) setzt man auf 1 Mol des Azolyl-Ketons der Formel (II) vorzugsweise 1 bis 2 Mol des Halogenierungsmittels ein.

Die Halogenvinyl-azol-Derivate der Formel (Ib)

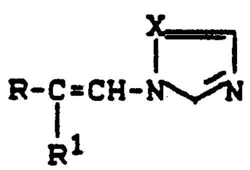

(Ib)

in welcher

R und X die oben angegebenen Bedeutungen haben und

$R^1$ für Halogen, wie Chlor oder Brom, steht,

können auch erhalten werden, indem man die Azolyl-Ketone der Formel (II) mit Triphenylphosphin und einem Hexahalogenethan, wie Hexachlorethan oder Hexabromethan, im Temperaturbereich von 60 °C bis 200 °C, vorzugsweise im Temperaturbereich von 80 °C bis 180 °C ohne Verdünnungsmittel umsetzt (vergl. hierzu auch die Herstellungsbeispiele).

Als Alkylierungsmittel bzw. Silylierungsmittel für die erfindungsgemäße Verfahrensvariante (b) verwendet man insbesondere Dialkylsulfate, wie z. B. Dimethylsulfat und Diethylsulfat, oder Trialkylsilylhalogenide, wie z. B. Trimethylsilylchlorid.

Als starke Basen kommen bei der Durchführung der erfindungsgemäßen Verfahrensvariante (b) alle üblichen starken anorganischen und organischen Basen in Frage. Hierzu gehören vorzugsweise Alkalihydride bzw. Alkaliamide, wie Natriumhydrid bzw. -amid und Kaliumhydrid bzw. -amid; tertiäre Alkylamine, wie Triethylamin; Alkalimetallalkoholate, wie Natriummethylat, Natriumethylat und Kalium-tert.-butylat; sowie Alkalihydroxide, wie Natriumhydroxid und Kaliumhydroxid.

Als Verdünnungsmittel kommen bei der Durchführung der erfindungsgemäßen Verfahrensvariante (b) alle üblichen inerten organischen Solventien in Betracht. Besonders geeignet sind Ether, wie Dioxan und Tetrahydrofuran; Formamide, wie Dimethylformamid; sowie Sulfoxide, wie Dimethylsulfoxid.

Die Reaktionstemperaturen können bei der Durchführung der erfindungsgemäßen Verfahrensvariante (b) in einem größeren Bereich variiert werden. Im allgemeinen arbeitet man bei Temperaturen zwischen 20 °C und 100 °C, vorzugsweise zwischen 40 °C und 80 °C.

Bei der Durchführung der erfindungsgemäßen Verfahrensvariante (b) setzt man auf 1 Mol des Azolyl-Ketons der Formel (II) vorzugsweise 1 Mol des Alkylierungsmittels ein.

Für die erfindungsgemäße Verfahrensvariante (c) kommen als starke Basen alle üblichen starken anorganischen und organischen Basen in Frage. Hierzu gehören vorzugsweise Alkalihydride bzw. Alkaliamide, wie Natriumhydrid bzw. -amid und Kaliumhydrid bzw. -amid; tertiäre Alkylamine, wie Triethylamin; Alkalimetallalkoholate, wie Natriummethylat, Natriumethylat und Kalium-tert.-butylat; sowie Alkalihydroxide, wie Natriumhydroxid und Kaliumhydroxid.

Als Verdünnungsmittel kommen bei der Durchführung der erfindungsgemäßen Verfahrensvariante (c) alle üblichen inerten organischen Solventien in Betracht. Auch hier sind besonders geeignet Ether, wie Dioxan und Tetrahydrofuran; Formamide, wie Dimethylformamid; Sulfoxide, wie Dimethylsulfoxid, sowie Alkohole, wie Ethanol.

Die Reaktionstemperaturen können bei der Durchführung der erfindungsgemäßen Verfahrensvariante (c) in einem größeren Bereich variiert werden. Im allgemeinen arbeitet man bei Temperaturen zwischen 0 °C und 120 °C, vorzugsweise zwischen 20 °C und 100 °C.

Bei der Durchführung der erfindungsgemäßen Verfahrenvariante (c) setzt man auf 1 Mol des Chlorvinyl-azol-Derivates der Formel (Ia) vorzugsweise 1 bis 2 Mol Base ein.

Die erfindungsgemäße Verfahrensvariante (d) wird unter einsatz von Halogenverbindungen der Formel (III) durchgeführt. In Formel (III) stehen $R^2$ und $R^3$ vorzugsweise für Chlor und/oder Brom, insbesondere für Chlor. Als Beispiele für die Halogenverbindungen der Formel (III) seien Chlor ($Cl_2$), Brom ($Br_2$) und "Bromchlorid" (BrCl) - letzteres gegebenenfalls in situ aus Brom und Chlor erzeugt - genannt.

Als Verdünnungsmittel kommen bei der Durchführung erfindungsgemäßen Verfahrensvariante (d) alle üblichen inerten organischen Solventien in Betracht. Besonders geeignet sind halogenierte Kohlenwasserstoffe, wie z. B. Methylenchlorid, 1,2-Dichlorethan, Chloroform, Tetrachlormethan, Chlorbenzol und Dichlorbenzole.

Die Reaktionstemperaturen können bei der erfindungsgemäßen Verfahrensvariante (d) in einem größeren Bereich variiert werden. Im allgemeinen arbeitet man bei Temperaturen zwischen 0 °C und 200 °C, vorzugsweise bei Temperaturen zwischen 20 °C und 150 °C.

Bei der Durchführung der erfindungsgemäßen Verfahrensvariante (d) setzt man auf 1 Mol Alkenyl-azol der Formel (Ib) vorzugsweise 1 bis 3 Mol einer Halogenverbindung der Formel (III) ein.

Für die erfindungsgemäße Verfahrensvariante (e) kommen als starke Basen alle üblichen starken anorganischen und organischen Basen in Frage. Hierzu gehören vorzugsweise Alkalihydride bzw. Alkaliamide, wie Natriumhydrid bzw. -amid und Kaliumhydrid bzw. -amid; tertiäre Alkylamine, wie Triethylamin; Alkalimetallalkoholate, wie Natriummethylat, Natriumethylat und Kalium-tert.-butylat; sowie Alkalihydroxide, wie Natriumhydroxid und Kaliumhydroxid.

Als Verdünnungsmittel kommen bei der Durchführung der erfindungsgemäßen Verfahrensvariante (e) alle üblichen inerten organischen Solventien in Betracht. Auch hier sind besonders geeignet Ether, wie Dioxan und Tetrahydrofuran; Formamide, wie Dimethylformamid; Sulfoxide, wie Dimethylsulfoxid, sowie Alkohole, wie Ethanol.

Die Reaktionstemperaturen können bei der Durchführung der erfindungsgemäßen Verfahrensvariante (e) in einem größeren Bereich variiert werden. Im allgemeinen arbeitet man bei Temperaturen zwischen 0 °C und 120 °C, vorzugsweise zwischen 20 °C und 100 °C.

Bei der Durchführung der erfindungsgemäßen Verfahrensvariante (e) setzt man auf 1 Mol des Halogenalkyl-azols der Formel (Ic) vorzugsweise 1 bis 2 Mol Base ein.

Zur Herstellung von Säureadditions-Salze der Verbindungen der allgemeinen Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise z. B. durch Abfiltrieren isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol, und Hinzufügen zu einer Lösung von Verbindungen der allgemeinen Formel (I). Man kann Metallsalz-Komplexe in bekannter Weise, z. B. durch Abfiltrieren, isolieren und gegebenenfalls durch Umkristallisation reinigen.

Als Photosynthesehemmer-Herbizid-Wirkstoffe für die oben bereits erwähnten Wirkstoffkombinationen seien vorzugsweise die folgenden Verbindungen der allgemeinen Formeln (VII-A) bis (VII-J) genannt:

(A) Triazinon-Derivate der Formel

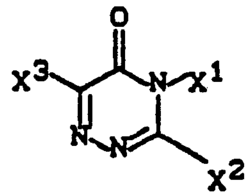

(VII-A)

in welcher
$X^1$ für Amino, gegebenenfalls substituiertes Alkylidenamino oder Alkyl mit 1 bis 2 Kohlenstoffatomen steht;
$X^2$ für Alkylthio mit 1 bis 2 Kohlenstoffatomen, Alkyl-und Dialkylamino mit jeweils 1 bis 2 Kohlenstoffatomen in jedem Alkylteil oder Alkyl mit 1 bis 4 Kohlenstoffatomen steht; und
$X^3$ für gegebenenfalls durch Halogen substituiertes tert.-Butyl oder gegebenenfalls substituiertes Phenyl steht.

(B) Triazindion-Derivate der Formel

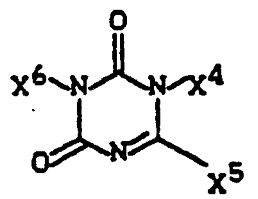

(VII-B)

in welcher
$X^4$ für Amino, gegebenenfalls substituiertes Al kylidenamino oder Alkyl mit 1 bis 2 Kohlenstoffatomen steht;
$X^5$ für Alkylthio mit 1 bis 2 Kohlenstoffatomen, Alkyl-und Dialkylamino mit jeweils 1 bis 2 Kohlenstoffatomen in jedem Alkylteil oder Alkyl mit 1 bis 4 Kohlenstoffatomen steht; und
$X^6$ für Alkyl mit 1 bis 6 Kohlenstoffatomen oder gegebenenfalls substituiertes Phenyl steht.

(C) Triazin-Derivate der Formel

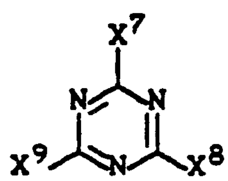

(VII-C)

in welcher
$X^7$ für Chlor, Alkoxy oder Alkylthio mit jeweils 1 bis 2 Kohlenstoffatomen steht;
$X^8$ für Alkylamino mit 1 bis 4 Kohlenstoffatomen im Alkylteil steht; und
$X^9$ für gegebenenfalls durch Cyano substituiertes Alkyl mit 1 bis 4 Kohlenstoffatomen steht.

(D) Harnstoff-Derivate der Formel

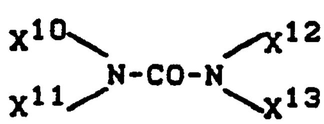

(VII-D)

in welcher
$X^{10}$ für gegebenenfalls substituiertes Phenyl, Benzthiazolyl oder gegebenenfalls substituiertes Thiadiazolyl steht;
$X^{11}$ für Wasserstoff oder Methyl steht;
$X^{12}$ für Methyl steht; und
$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht.

(E) Carboxanilid-Derivate der Formel

$X^{14}$-CO-NH-$X^{15}$ (VII-E)

in welcher
$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht.

(F) Uracil-Derivate der Formel

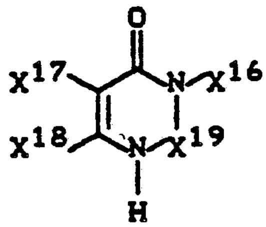

(VII-F)

in welcher

$X^{16}$ für Alkyl mit 1 bis 6 Kohlenstoffatomen oder cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die CO-oder $SO_2$-Gruppe steht.

(G) Biscarbamat-Derivate der Formel

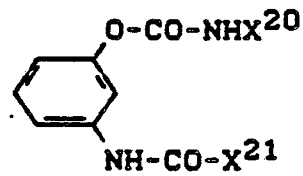

(VII-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder dialkylamino mit 1 bis 2 Kohlenstoffatomen in jedem Alkylteil steht.

(H) Pyridazinon-Derivate der Formel

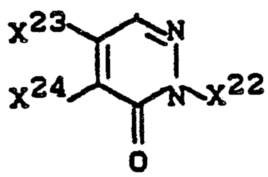

(VII-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht.

(J) Hydroxybenzonitril-Derivate der Formel

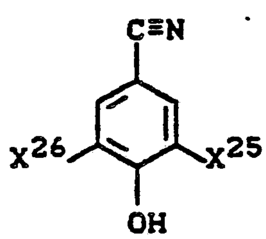

(VII-J)

in welcher

$X^{25}$ für Halogen steht; und

$X^{26}$ für Halogen steht.

Besonders bevorzugt sind folgende Photosynthesehemmer-Wirkstoffe der allgemeinen Formeln (VII-A) bis (VII-J):

(A) Triazinon-Derivate der Formeln

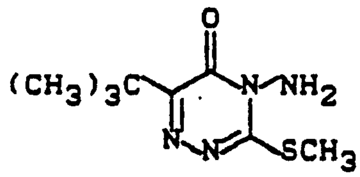

(VII-A-1)
(Metribuzin)

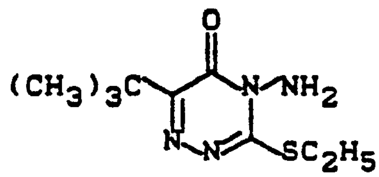

(VII-A-2)

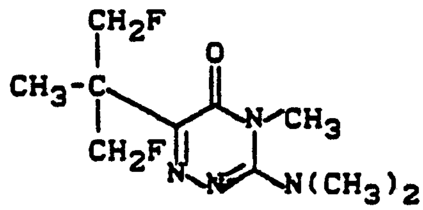

(VII-A-3)

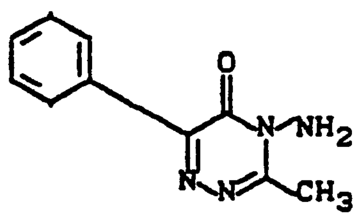

(VII-A-4)
(Metamitron)

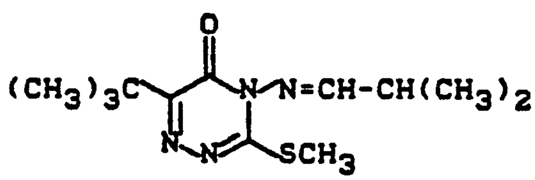

(VII-A-5)
(Isomethiozin)

(B) Triazindion-Derivat der Formel

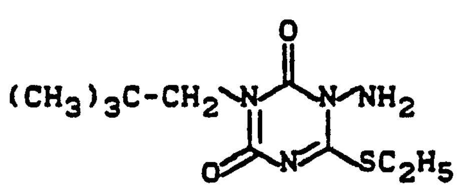

(VII-B-1)
(Ametridione)

(C) Triazin-Derivate der Formeln

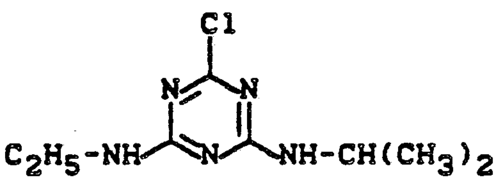

(VII-C-1)
(Atrazin)

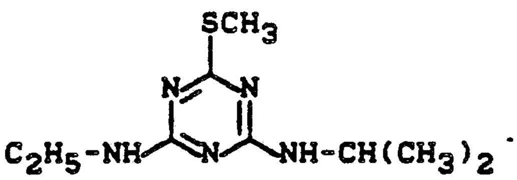

(VII-C-2)
(Ametryn)

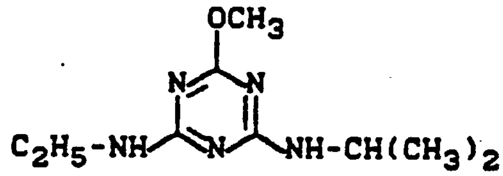

(VII-C-3)
(Atraton)

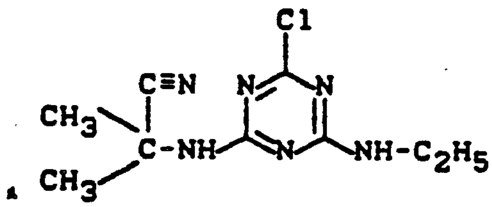

(VII-C-4)
(Cyanazin)

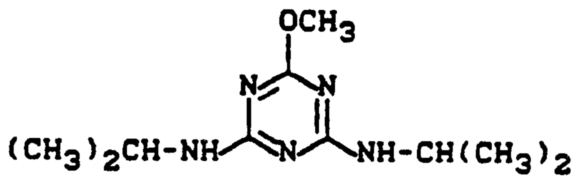

(VII-C-5)
(Prometon)

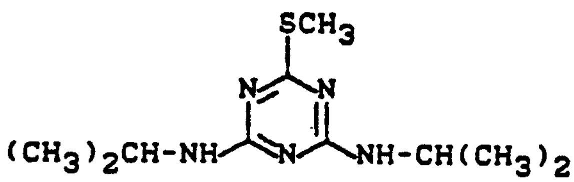

(VII-C-6)
(Prometryn)

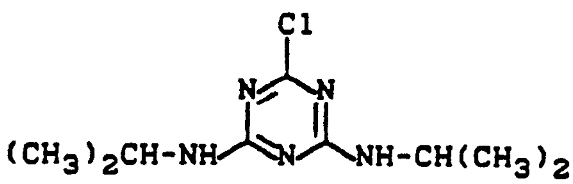

(VII-C-7)
(Propazin)

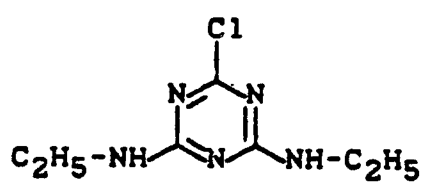

(VII-C-8)
(Simazin)

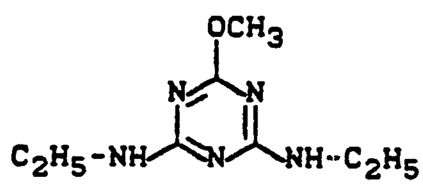

(VII-C-9)
(Simeton)

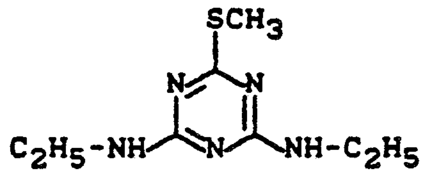

(VII-C-10)
(Simetryn)

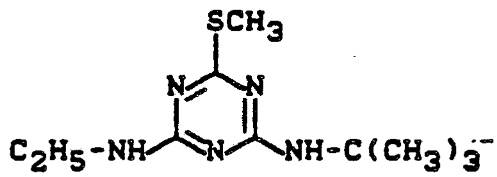

(VII-C-11)
(Terbutryn)

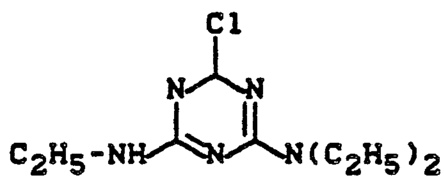

(VII-C-12)
(Trietazin)

(D) Harnstoff-Derivate der Formeln

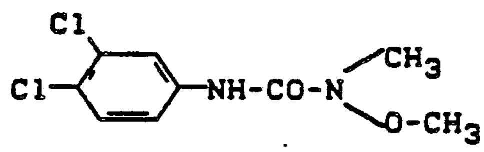

(VII-D-1)
(Linuron)

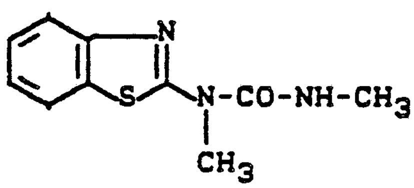

(VII-D-2)
(Methabenzthiazuron)

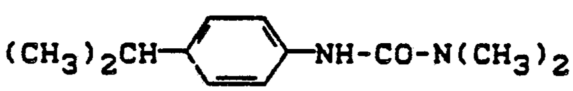

(VII-D-3)
(Isoproturon)

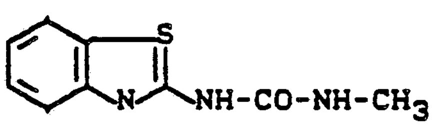

(VII-D-4)
(Benzthiazuron)

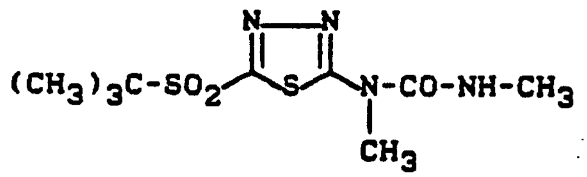

(VII-D-5)
(Buthiuron)

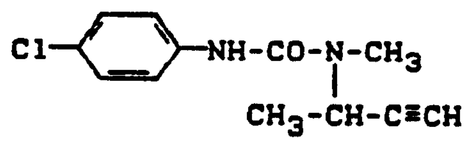

(VII-D-6)
(Buturon)

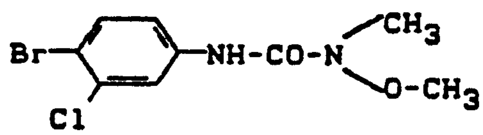

(VII-D-7)
(Chlorbromuron)

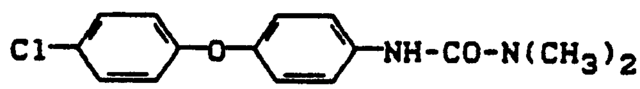

(VII-D-8)
(Chloroxuron)

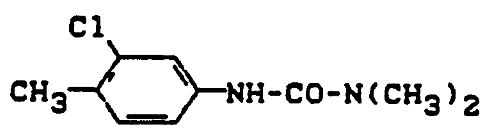

(VII-D-9)
(Chlortoluron)

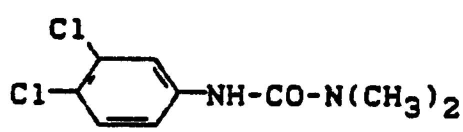

(VII-D-10)
(Diuron)

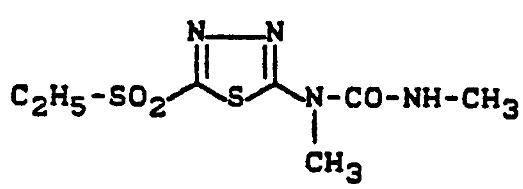

(VII-D-11)
(Ethidimuron)

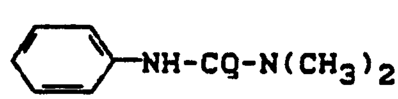

(VII-D-12)
(Fenuron)

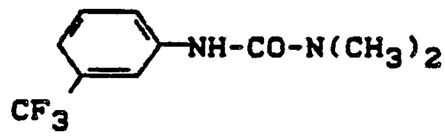

(VII-D-13)
(Fluometuron)

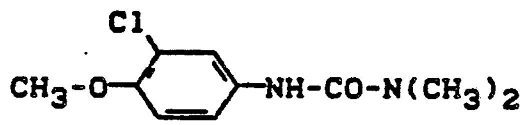

(VII-D-14)
(Metoxuron)

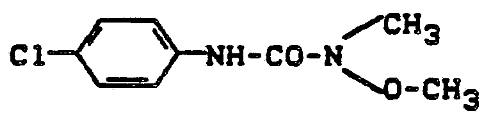

(VII-D-15)
(Monolinuron)

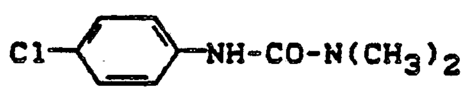

(VII-D-16)
(Monuron)

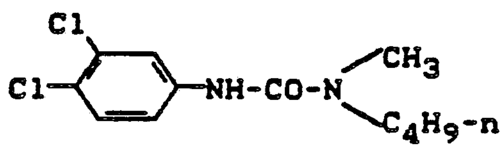

(VII-D-17)
(Neburon)

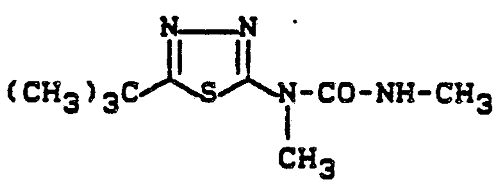

(VII-D-18)
(Tebuthiuron)

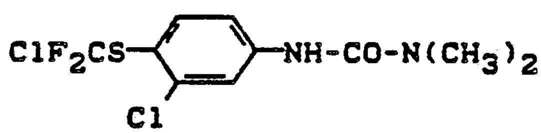

(VII-D-19)
(Thiochlormethyl)

(E) Carboxanilid-Derivate der Formeln

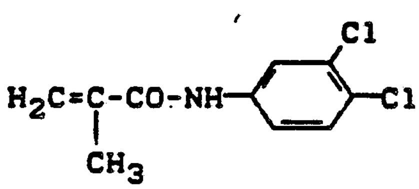

(VII-E-1)
(Chloranocryl)

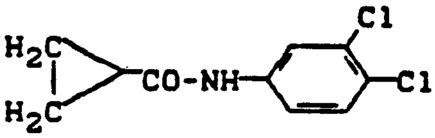

(VII-E-2)
(Cypromid)

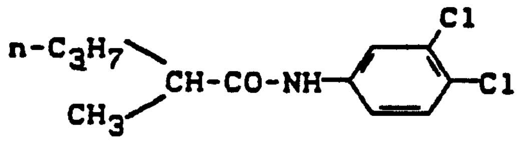

(VII-E-3)
(Karsil)

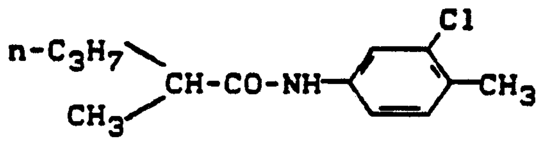

(VII-E-4)
(Pentanochlor)

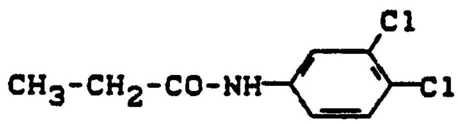

(VII-E-5)
(Propanil)

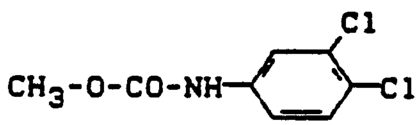

(VII-E-6)
(Swep)

(F) Uracil-Derivate der Formeln

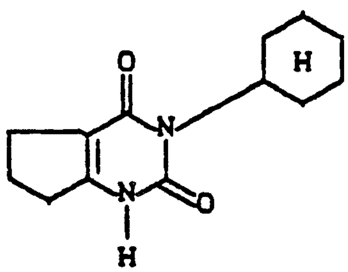

(VII-F-1)
(Lenacil)

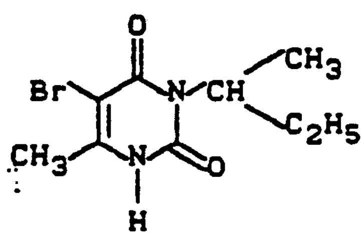

(VII-F-2)
(Bromacil)

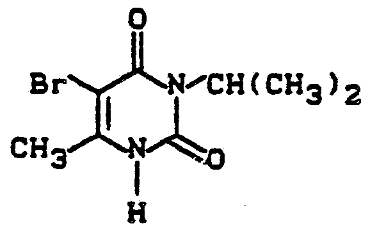

(VII-F-3)
(Isocil)

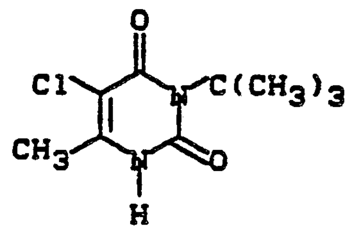

(VII-F-4)
(Terbacil)

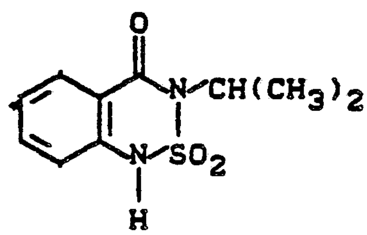

(VII-F-5)
(Bentazon)

(G) Biscarbamat-Derivate der Formeln

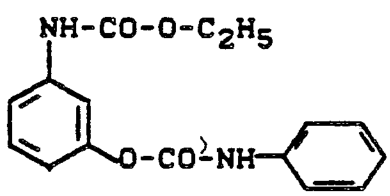

(VII-G-1)
(Desmedipham)

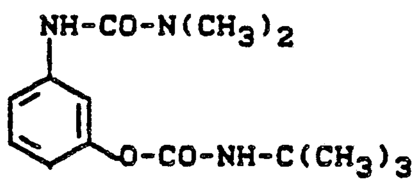

(VII-G-2)
(Karbutilate)

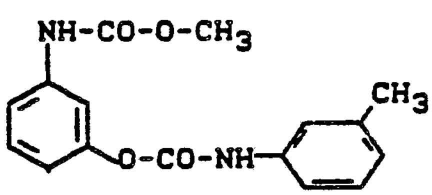

(VII-G-3)
(Phenmedipham)

(H) Pyridazinon-Derivate der Formeln

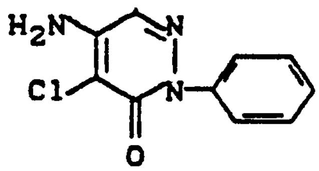

(VII-H-1)
(Pyrazon)

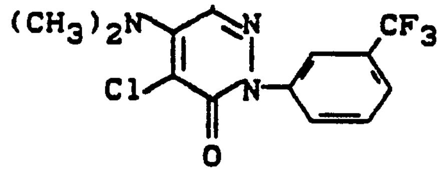

(VII-H-2)
(Metflurazon)

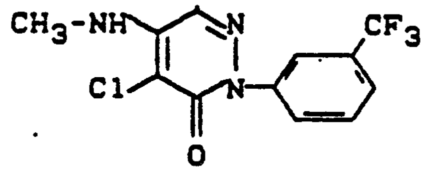

(VII-H-3)
(Norflurazon)

(J) Hydroxybenzonitril-Derivate der Formeln

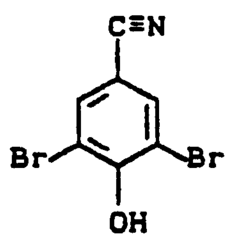

(VII-J-1)
(Bromoxynil)

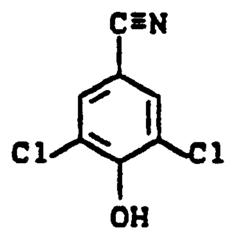

(VII-J-2)
(Chloroxynil)

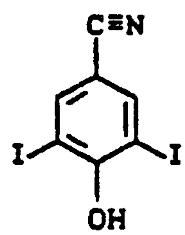

(VII-J-3)
(Ioxynil)

Die Photosynthesehemmer-Wirkstoffe der Formeln (VII-A) bis (VII-J) sind bekannt (vergl. z. B. Carl Fedtke, Biochemistry and Physiology of Herbicide Action, Springer-Verlag, 1982).

Die Gewichtsverhältnisse der Wirkstoffe in den neuen Wirkstoffkombinationen können in relativ großen Bereichen schwanken. Im allgemeinen entfallen auf ein Gewichtsteil Photosynthesehemmer-Wirkstoff (herbizider Wirkstoff) 0,25 bis 100, vorzugsweise 5 bis 50, insbesondere 10 bis 20, Gew.-Teile Verbindung der Formel (I) (Synergist).

Die Photosynthesehemmer-Wirkstoffe weisen starke herbizide Wirkungen auf. Dennoch besitzen sie gegen einige Unkräuter, wie z. B. Galium aparine, Iopmoea hederacea, Datura stramonium, Cirsium arvense, Convolvulus arvensis oder Solanum nigrum und einige Ungräser, wie z. B. Agropyron repens,

Avena fatua, Cynodon dactylon, Cyperus ssp. und Lolium rigidum eine nicht immer ausreichende Wirkung. Die erfindungsgemäßen Wirkstoffkombinationen dehnen das Wirkungsspektrum der Verbindungen der Formeln (VII-A) bis (VII-J) aus und ermöglichen dadurch eine Bekämpfung dieser durch die herbiziden Wirkstoffe alleine nur schwer oder gar nicht bekämpfbaren Unkräuter.

Die erfindungsgemäßen Wirkstoffkombinationen aus Photosynthesehemmer-Herbiziden und Verbindungen der Formel (I) können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dac tyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen zeigen insbesondere neben einer guten Wirkung gegen grasartige (monokotyle) Unkräuter auch eine gute herbizide Wirkung bei dikotylen Unkräutern.

Die neuen Wirkstoffkombinationen zur Unkrautbekämpfung können als solche oder in ihren Formulierungen auch in Mischung mit anderen bekannten Herbiziden zur Unkrautbekämpfung Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffkombinationen zur Unkrautbekämpfung können sowohl vor als auch nach der Einsaat ebenso wie nach dem Auflaufen der Pflanzen gemeinsam oder in getrennten Anwendungen ausgebracht werden. Hierbei spielt die Reihenfolge der Anwendung keine Rolle.

Beim Einsatz der erfindungsgemäßen Synergisten kann die übliche Aufwandmenge des Herbizids der Formeln (VII-A) bis (VII-J) verringert werden. Die Aufwandmenge an her bizidem Photosynthesehemmer-Wirkstoff liegt bei Flächenbehandlung zwischen 0,01 und 3,0 kg/ha, vorzugsweise zwischen 0,05 und 2,0 kg/ha. Die Aufwandmenge an synergistischen Verbindungen der Formel (I) liegt bei Flächenbehandlung zwischen 0,1 und 10 kg/ha, vorzugsweise zwischen 0,5 und 3 kg/ha.

Die gute herbizide Wirkung der erfindungsgemäßen Wirkstoffkombinationen geht aus Verwendungsbeispiel (A) hervor. Während die einzelnen Wirkstoffe in der herbiziden Wirkung Schwächen aufweisen, zeigen die Kombinationen eine Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die Summe der Wirkungen der einzelnen applizierten Wirkstoffe.

Die erfindungsgemäßen Wirkstoffkombinationen aus Arthropodiziden und Verbindungen der Formel (I) und deren Salzen sowie die arthropodiziden Schädlingsbekämpfungsmittel, welche diese Wirkstoffkombinationen enthalten, zeigen nicht nur eine schnelle knock-down-Wirkung, sondern bewirken auch die Abtötung der tierischen Schädlinge, vorzugsweise von Arthropoden, insbesondere Insekten und Spinnentieren (einschließlich Milben), die in der Landwirtschaft (einschließlich der Tierhaltung), in Forsten, im Vorrats-und Materialschutz sowie auf dem Haushalt-und Hygienesektor vorkommen. Sie sind gegen normal sensible und resistente Arten sowie gegen alle oder einzelne Entwicklungsstadien wirksam.

Zu den tierischen Schädlingen, welche unter Verwendung der Verbindungen der Formel (I) und deren Salzen bekämpft werden können, gehören beispielsweise:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.b. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffkombinationen aus den Verbindungen der Formel (I) und deren Salzen und den übrigen Wirkstoffen können in Abhängigkeit von ihren jeweiligen physikalischen und/oder chemischen Eigenschaften in übliche Formulierungen übergeführt werden, die Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesent lichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Als weitere Zusätze können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der gesamte Wirkstoffgehalt (einschließlich Synergist) der aus den handelsüblichen Formulierungen bereiteten Anwendungsformen kann in weiten Bereichen variieren. Die Wirkstoffkonzentration der Anwendungsformen kann von 0,001 bis zu 95 Gew.-% Wirkstoffkombination, vorzugsweise zwischen 0,01 und 10 Gew.-% liegen.

Die Anwendung geschieht in einer den Anwendungsformen angepaßten üblichen Weise.

Bei der Anwendung gegen Hygiene-und Vorratsschädlinge zeichenen sich die Wirkstoffkombinationen zur Bekämpfung von Arthropoden durch eine hervorragende Residualwirkung auf Holz und Ton sowie durch eine gute Alkalistabilität auf gekalten Unterlage aus.

Die Gewichtsverhältnisse der Synergisten der allgemeinen Formel (I) bzw. deren Salze und Wirkstoffe (Arthropodizide bzw. Herbizide) können in einem relativ großen Bereich variiert werden. Im allgemeinen werden die als Synergisten verwendeten Verbindungen der Formel (I) bzw. deren Salze mit den übrigen Wirkstoffen in Mischungsverhältnissen zwischen 1 : 100 und 100 : 1, vorzugsweise zwischen 1 : 20 und 20 : 1, insbesondere zwischen 1 : 10 und 10 : 1 (Gewichtsteile) eingesetzt.

Die gute arthropodizide Wirkung der erfindungsgemäßen Wirkstoffkombinationen aus Verbindungen der Formel (I) bzw. deren Salze und Arthropodiziden geht aus den Verwendungsbeispielen (B) und (C) hervor.

Neben den angegebenen Eigenschaften zeigen die erfindungsgemäßen Verbindungen der Formel (I) auch eine wachstumsregulierende Wirkung.

Das erfindungsgemäße Herstellungsverfahren soll anhand der folgenden Herstellungsbeispiele erläutert werden:

Beispiel 1

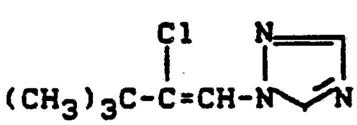

(Verfahrensvariante a)

Eine Lösung von 50 g (0,29 Mol) 3,3-Dimethyl-1-(1,2,4-triazol-1-yl)-butan-2-on in 100 ml Phosphoroxytrichlorid wird unter Rühren mit 93 g (0,45 Mol) Phosphorpentachlorid versetzt. Das Reaktionsgemisch wird 2 Stunden auf 100 °C erwärmt, anschließend auf Eis gegossen, mit konzentrierter Natronlauge auf pH 8 eingestellt und mit Dichlormethan extrahiert. Nach Entfernen des Dichlormethans am Rotationsverdampfer wird der Rückstand destilliert.

Man erhält 42 g (76 % der Theorie) 2-Chlor-3,3-dimethyl-1-(1,2,4-triazol-1-yl)-but-1-en vom Siedepunkt 77 °C/0,08 mbar.

(Zusätzliche Verfahrensvariante):

396 g (1,7 Mol) Hexachlorethan und 437 g (1,7 Mol) Triphenylphosphin werden zusammen mit 284 g (1,7 Mol) 3,3-Dimethyl-1-(1,2,4-triazol-1-yl)-butan-2-on langsam erwärmt. Bei 70 °C Innentemperatur tritt eine exotherme Reaktion ein, wobei die Temperatur auf 115 °C steigt.

Das Reaktionsgemisch wird im Ölbad auf 170 °C erhitzt und 6 Stunden bei 170 °C gerührt. Nach dem Erkalten wird das dunkle Produkt in 1,5 l Dichlormethan gelöst und mit 2 l Wasser versetzt. Unter Umrühren wird das Gemisch mit Kaliumcarbonat auf pH 6 - 7 eingestellt, die organische Phase wird abgtrennt und am Rotationsverdampfer eingeengt. Der Rückstand wird mit heißem Petrolether (3 mal je 1 l) extrahiert und das Extrakt eingedampft. Das verbleibende Öl wird destilliert.

Man erhält 221 g (70 % der Theorie) 2-Chlor-3,3-dimethyl-1-(1,2,4-triazol-1-yl)-but-1-en vom Siedepunkt 85 °C/0,12 mbar.

Beispiel 2

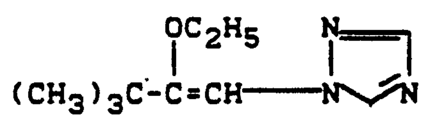

(Verfahrensvariante b)

Zu einer Lösung von 50 g (0,29 Mol) 3,3-Dimethyl-1-(1,2,4-triazol-1-yl)-butan-2-on in 200 ml Dimethylformamid werden vorsichtig 9 g (0,3 Mol) 80 prozentiges Natriumhydrid hinzugefügt. Nach 10 Minuten Rühren werden 46,2 g (0,3 Mol) Diethylsulfat bei 30 - 40 ° C zugetropft und das Reaktionsgemisch wird 2 Stunden bei 60 °C gerührt; anschließend wird das Gemisch in Wasser gegossen. Das Produkt wird mit Dichlormethan extrahiert, das Lösungsmittel am Rotationsverdampfer entfernt und der Rückstand über eine Vigreux-Kolonne destilliert.

Man erhält 16 g (28 % der Theorie) 2-Ethoxy-3,3-dimethyl-1-(1,2,4-triazol-1-yl)-but-1-en vom Siedepunkt 85 - 90 °C/0,665 mbar.

Beispiel 3

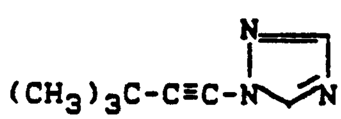

(Verfahrensvariante c)

Zu einer Lösung von 3 g (0,13 Mol) Natrium in 100 ml Ethanol gibt man bei 20 °C 18,6 g (0,1 Mol) 3,3-Dimethyl-1-(1,2,4-triazol-1-yl)-2-chlor-but-1-en und erwärmt 1 Stunde am Rückfluß. Anschließend entfernt man das überschüssige Ethanol am Rotationsverdampfer, gießt den Rückstand in Wasser und extrahiert

das Produkt mit Dichlormethan. Nach dem Entfernen des Lösungsmittels wird das zurückbleibende Öl destilliert.

Man erhält 11 g (74 % der Theorie) 3,3-Dimethyl-1-(1,2,4-triazol-1-yl)-but-1-in vom Siedepunkt 150 °C/0,266 mbar.

Beispiel 4

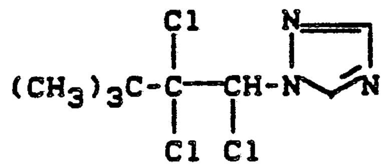

(Verfahrensvariante d)

In eine siedende Lösung aus 55,7 g (0,3 Mol) 2-Chlor-3,3-dimethyl-1-(1,2,4-triazol-1-yl)-buten und 200 ml Chlorbenzol werden innerhalb von ca. 45 Minuten 42 g (0,6 Mol) Chlor eingeleitet. Nach dem Abkühlen wird vom ausgefallenen Feststoff abfiltriert und das Filtrat wird am Rotationsverdampfer eingeengt. Der zurückbleibende ölige Rückstand wird unter vermindertem Druck destilliert.

Man erhält 49,6 g (64 % der Theorie) 3,3-Dimethyl-1-(1,2,4-triazol-1-yl)-1,2,2-trichlor-butan vom Siedepunkt 88 °C bis 94 °C/0,1 mbar.

Beispiel 5

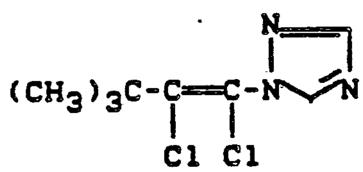

(Verfahrensvariante e)

1,3 g (0,055 Mol) Natrium werden zu 80 ml Ethanol gegeben. Dann werden 12,8 g (0,05 Mol) 3,3-Dimethyl-1-(1,2,4-triazol-1-yl)-1,2,2-trichlor-butan dazu gegeben und das Reaktionsgemisch wird ca. 15 Stunden bei 50 °C gerührt. Nach Einengen am Rotationsverdampfer wird der Rückstand zwischen Methylenchlorid und Wasser verteilt und die organische Phase wird abgetrennt. Nach Trocknen mit Natriumsulfat wird filtriert, das Filtrat eingeengt und der Rückstand unter vermindertem Druck destilliert.

Man erhält 7,1 g (65 % der Theorie) 1,2-Dichlor-3,3-dimethyl-1-(1,2,4-triazol-1-yl)-buten vom Siedepunkt 87 °C bis 93 °C/0,4 mbar.

In analoger Weise und entsprechend den erfindungsgemäßen Verfahrensvarianten werden die nachfolgenden Verbindungen der allgemeinen Formel (I)

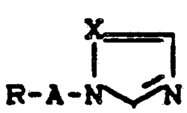

(I)

erhalten:

| Beisp.-Nr. | R | A | X | Siedepunkt (°C/mbar) |
|---|---|---|---|---|
| 6 | $(CH_3)_3C-$ | 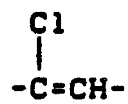 | -CH- | 88/0,2 |
| 7 | $(CH_3)_3C-$ | 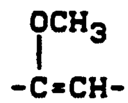 | N | 74/0,133 |
| 8 | 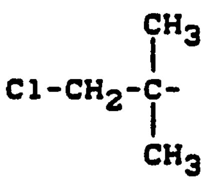 | 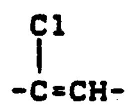 | N | 130/0,266 |
| 9 | 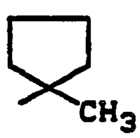 | 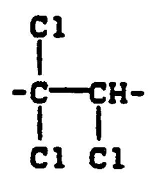 | N | [Fp: 95°C] |

| Beisp.-Nr. | R | A | X | Siedepunkt (°C/mbar) |
|---|---|---|---|---|
| 10 | 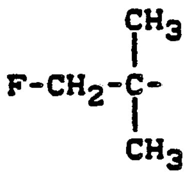 | 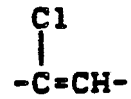 | N | 75/0,8 |
| 11 | $(CH_3)_3C-$ | 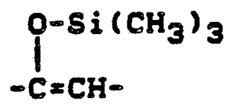 | N | 80/0,266 |
| 12 | 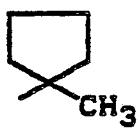 | 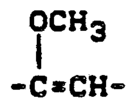 | N | 125/0,532 |
| 13 | 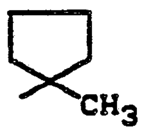 | 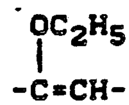 | N | 126/0,266 |
| 14 | 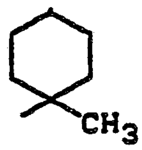 | 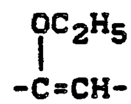 | N | 128/0,2 |
| 15 | 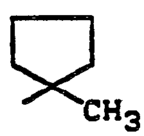 | 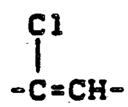 | N | 103/0,133 |
| 16 | 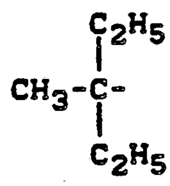 | 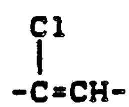 | N | 84/0,133 |
| 17 | 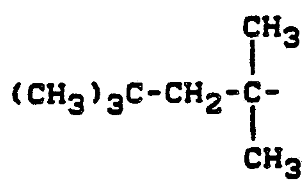 | 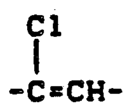 | N | 100/0,133 |

Verwendungsbeispiel A (Herbizid-Synergismus)

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil herbiziden Wirkstoffs bzw. Synergisten bzw. eines Gemisches aus herbizidem Wirkstoff und Synergisten mit der

angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit einer Herbizid-Zubereitung bzw. mit der Synergisten-Zubereitung bzw. mit der Zubereitung aus Synergisten und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle A hervor.

Pre-emergence-Test

**Tabelle A**

**Synergistische Wirkung von Verbindungen der Formel (I) (=Synergist S) und 4-Amino-6-tert.-butyl-3-methylthio-1,2,4-triazin-5-on (VII-A-1) (= Herbizid H) an Ipomoea hederacea. Die Aufwandmenge in kg/ha bezieht sich auf den Gehalt an Wirkstoff.**

| Struktur des Synergisten (III) | | (S) kg/ha | (H) kg/ha | % Schäden bei Ipomoea hederacea H | S | H + S |
|---|---|---|---|---|---|---|
| (1) | 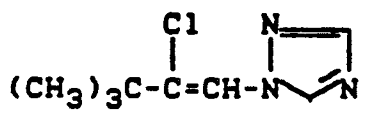 | 0,3 | 0,07 | 10 | - | 90 |
| | | 1,0 | 0,07 | 10 | 0 | 70 |
| (6) | 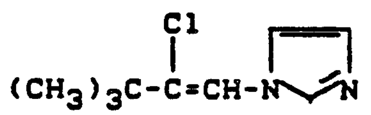 | 0,3 | 0,1 | 10 | - | 30 |
| | | 2,0 | 0,1 | 10 | 0 | 100 |
| (3) | 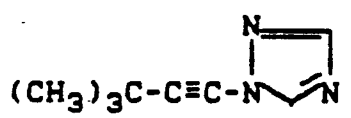 | 0,3 | 0,1 | 10 | - | 80 |
| | | 2,0 | 0,1 | 10 | 10 | 100 |
| (8) | 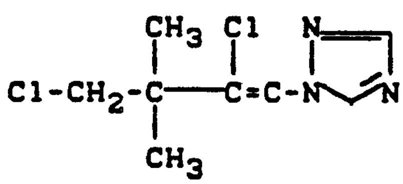 | 0,3 | 0,1 | 10 | - | 90 |
| | | 2,0 | 0,1 | 10 | 0 | 90 |

## Tabelle A (Fortsetzung)

| Struktur des Synergisten | (S) kg/ha | (H) kg/ha | % Schäden bei Ipomoea hederacea H | S | H + S |
|---|---|---|---|---|---|
| (7) 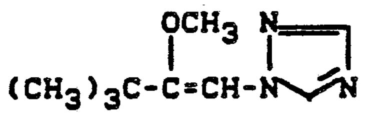 | 0,3 | 0,1 | 10 | - | 50 |
| | 2,0 | 0,1 | 10 | 0 | 60 |
| (2) 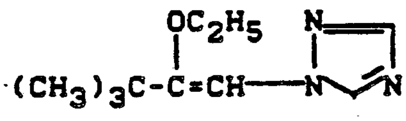 | 0,3 | 0,1 | 10 | - | 30 |
| | 2,0 | 0,1 | 10 | 0 | 60 |
| (10) 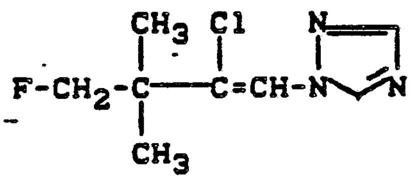 | 0,3 | 0,1 | 10 | - | 90 |
| | 2,0 | 0,1 | 10 | 0 | 100 |

In den nachfolgenden Verwendungsbeispielen (B) und (C) soll die synergistische Wirksamkeit der erfindungsgemäßen Verbindungen der Formel (I) in Kombination mit Arthropodiziden erläutert werden.

Beispiele für die erfindungsgemäß verwendbaren Arthropodizide sind nachstehend aufgeführt:

(A) 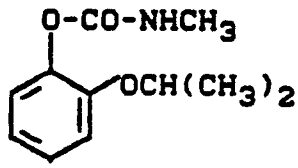 (Propoxur)

(B) 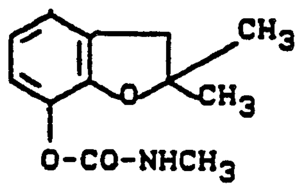 (Carbofuran)

(C) 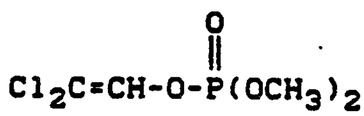 (DDVP)

(D) 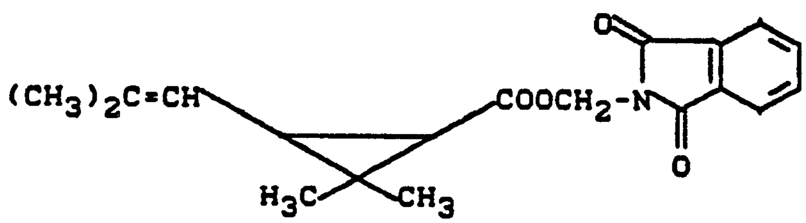

(Tetramethrin)

(E) 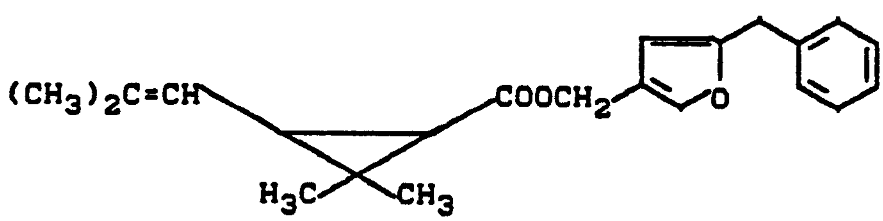

(Resmethrin)

(F) 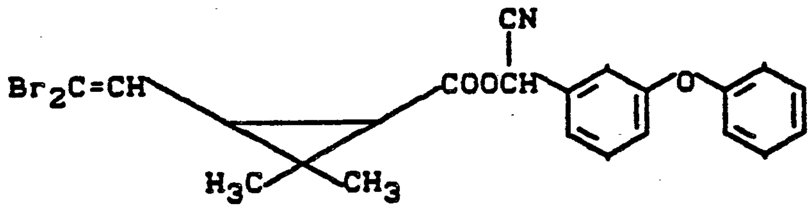

(Deltamethrin)

(G) 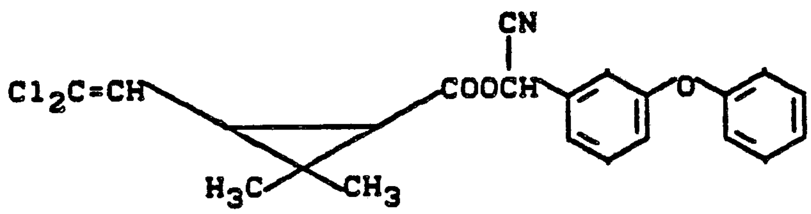

(Cypermethrin)

(H) 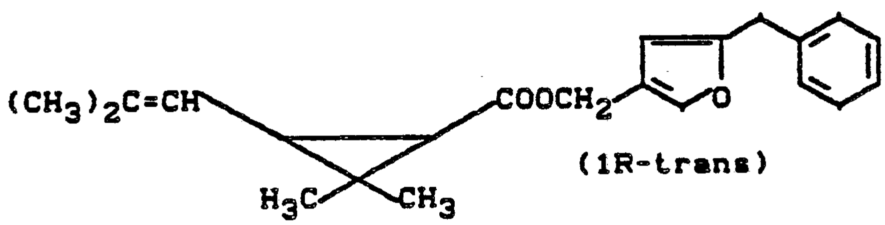

(1R-trans)

(Bioresmethrin)

(I) 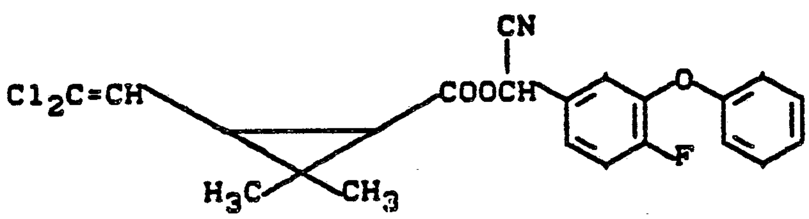

(Cyfluthrin)

Beispiele für die erfindungsgemäß verwendbaren Insektizid-Synergisten sind nachstehend aufgeführt:

| Synergist Nr. | Formel | Verbindung aus Herstellungsbeispiel Nr. |
|---|---|---|
| (1) | 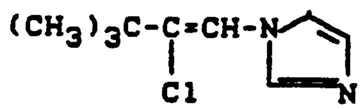 | 6 |
| (2) | 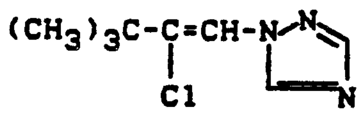 | 1 |
| (3) | 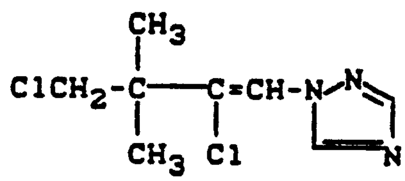 | 8 |
| (4) | 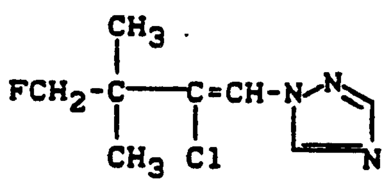 | 10 |
| (5) | 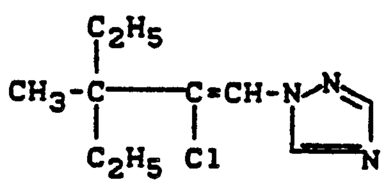 | 16 |
| (6) | 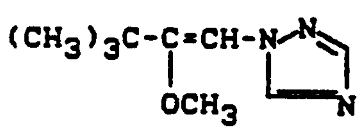 | 7 |
| (7) | 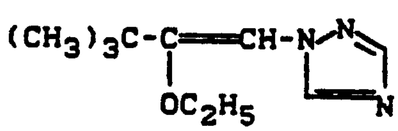 | 2 |

| Synergist Nr. | Formel | Verbindung aus Herstellungs-beispiel Nr. |
|---|---|---|
| (8) | 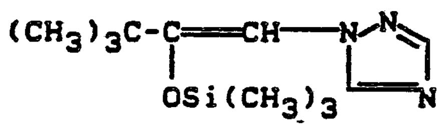 | 11 |
| (9) | 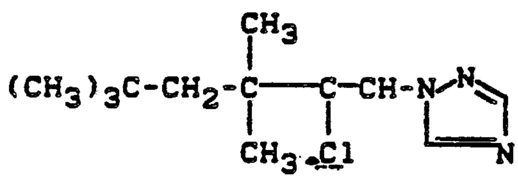 | 17 |
| (10) | 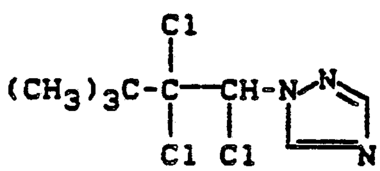 | 4 |
| (11) | 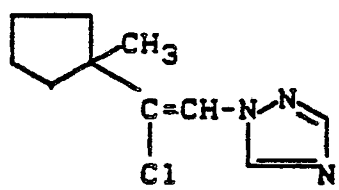 | 15 |
| (12) | 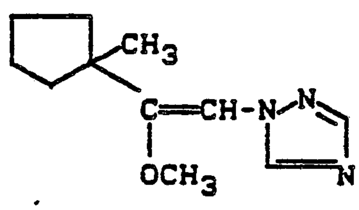 | 12 |
| (13) | 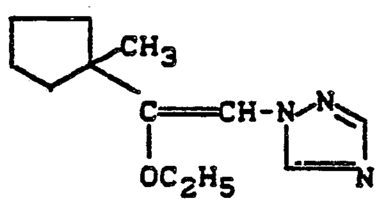 | 13 |

| Synergist Nr. | Formel | Verbindung aus Herstellungsbeispiel Nr. |
|---|---|---|
| (14) | 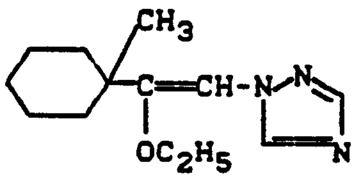 | 14 |

Verwendungsbeispiel B (Insektizid-Synergismus)

$KT_{50}$-Aerosoltest

Testtiere: Musca domestica ♂♂, Stamm Weymanns (gegen Carbamate und Phosphorsäureester resistent)
Lösungsmittel: Aceton

Zur Herstellung einer zweckmäßigen Wirkstoffzubereitung löst man die Wirkstoffe, Synergisten und Wirkstoff/Synergisten-Gemische, in dem Lösungsmittel.

In die Mitte einer gasdichten Glaskammer von 1 $m^3$ Größe werden drei Drahtkäfige gehängt, in denen sich je 20 Versuchstiere befinden. Nachdem die Kammer wieder verschlossen wurde, werden in ihr 2 ml der Wirkstoffzubereitung zerstäubt. Der Zustand der Testtiere wird durch die Glaswände von außen ständig kontrolliert und diejenige Zeit ermittelt, die für eine 50 %ige knock down-Wirkung ($KT_{50}$) der Tiere notwendig ist. Wird nach 60 Minuten keine $KT_{50}$ erreicht, wird der %-Satz der knock down gegangenen Tiere bestimmt.

Wirkstoffe, Wirkstoffmengen und Zeiten, bei denen eine 50 %ige knock down-Wirkung vorliegt und der %-Satz der nach 60 Minuten knock down (K.D.) gegangenen Tiere gehen aus der nachfolgenden Tabelle hervor:

## Tabelle B

**$KT_{50}$-Aerosoltest mit gegen Phosphorsäureester- und Carbamate-resistenten männlichen Musca domestica (Stamm Weymanns)**

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| A | 20 | Keine (60' = 2 %) |
| B | 10 | Keine (60' = 25 %) |
| C | 10 | 23' |
| | 5 | 36' |
| 1 | 20 | Keine (60' = 0 %) |
| 2 | 20 | Keine (60' = 0 %) |
| 3 | 20 | Keine (60' = 0 %) |
| 4 | 20 | Keine (60' = 0 %) |
| 5 | 20 | Keine (60' = 0 %) |
| 6 | 20 | Keine (60' = 0 %) |
| 7 | 20 | Keine (60' = 0 %) |
| 8 | 20 | Keine (60' = 0 %) |
| 9 | 20 | Keine (60' = 0 %) |

**Tabelle B - Fortsetzung**

$KT_{50}$-Aerosoltest mit gegen Phosphorsäureester- und Carbamate-resistenten männlichen Musca domestica (Stamm Weymanns)

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| 10 | 20 | Keine (60' = 0 %) |
| 11 | 20 | Keine (60' = 0 %) |
| 12 | 20 | Keine (60' = 0 %) |
| 13 | 20 | Keine (60' = 0 %) |
| 14 | 20 | Keine (60' = 0 %) |
| A + 1 | 10 + 10 | 45' |
| A + 2 | 10 + 10 | 33' |
| A + 3 | 10 + 10 | 36' |
| A + 4 | 10 + 10 | 31' |
| A + 5 | 10 + 10 | 33' |
| A + 6 | 10 + 10 | 38' |
| A + 7 | 10 + 10 | 47' |
| A + 8 | 10 + 10 | 49' |

**Tabelle B - Fortsetzung**

$KT_{50}$-Aerosoltest mit gegen Phosphorsäureester- und Carbamate-resistenten männlichen Musca domestica (Stamm Weymanns)

| Wirkstoffe / Synergisten | | | Konzentration in $mg/m^3$ Wirkstoffe + Synergisten | | | $KT_{50}$ innerhalb v. 60 Minuten (% K. D. nach 60 Minuten) |
|---|---|---|---|---|---|---|
| A | + | 9 | 10 | + | 10 | 43' |
| A | + | 10 | 10 | + | 10 | 35' |
| A | + | 11 | 10 | + | 20 | 36' |
| A | + | 11 | 10 | + | 10 | 36' |
| A | + | 11 | 10 | + | 8 | 37' |
| A | + | 11 | 10 | + | 5 | 37' |
| A | + | 11 | 10 | + | 2 | 38' |
| A | + | 11 | 10 | + | 1 | 46' |
| A | + | 12 | 10 | + | 10 | 32' |
| A | + | 13 | 10 | + | 10 | 33' |
| A | + | 14 | 10 | + | 10 | 38' |
| B | + | 11 | 10 | + | 10 | 22' |
| B | + | 11 | 10 | + | 5 | 22' |

**Tabelle B** - Fortsetzung

$KT_{50}$-Aerosoltest mit gegen Phosphorsäureester- und Carbamate-resistenten männlichen Musca domestica (Stamm Weymanns)

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| B + 11 | 10 + 2 | 26' |
| B + 11 | 10 + 1 | 26' |
| C + 11 | 10 + 20 | 13' |
| C + 11 | 10 + 10 | 15' |
| C + 11 | 10 + 5 | 15' |

Verwendungsbeispiel C (Insektizid-Synergismus)

$KT_{50}$-Aerosoltest

Testtiere: Musca domestica ♂♂, Stamm Hans (gegen Pyrethroide resistent)
Lösungsmittel: Aceton

Zur Herstellung einer zweckmäßigen Wirkstoffzubereitung löst man die Wirkstoffe, Synergisten und Wirkstoff/Synergisten-Gemische, in dem Lösungsmittel.

In die Mitte einer gasdichten Glaskammer von 1 $m^3$ Größe werden drei Drahtkäfige gehängt, in denen sich je 20 Versuchstiere befinden. Nachdem die Kammer wieder verschlossen wurde, werden in ihr 2 ml der Wirkstoffzubereitung zerstäubt. Der Zustand der Testtiere wird durch die Glaswände von außen ständig kontrolliert und diejenige Zeit ermittelt, die für eine 50 %ige knock down-Wirkung ($KT_{50}$) der Tiere notwendig ist. Wird nach 60 Minuten keine $KT_{50}$ erreicht, wird der %-Satz der knock down gegangenen Tiere bestimmt.

Wirkstoffe, Wirkstoffmengen und Zeiten, bei denen eine 50 %ige knock down-Wirkung vorliegt und der %-Satz der nach 60 Minuten knock down (K.D.) gegangenen Tiere gehen aus der nachfolgenden Tabelle hervor:

**Tabelle C**

$KT_{50}$-Aerosoltest mit gegen Pyrethroide resistenten männlichen Musca domestica (Stamm Hans)

| Wirkstoffe / Synergisten | | Konzentration in $mg/m^3$ Wirkstoffe + Synergisten | | $KT_{50}$ innerhalb v. 60 Minuten (% K. D. nach 60 Minuten) | |
|---|---|---|---|---|---|
| D | | 10 | | Keine | (60' = 0 %) |
| E | | 20 | | Keine | (60' = 30 %) |
| F | | 4 | | Keine | (60' = 22 %) |
| G | | 4 | | Keine | (60' = 0 %) |
| H | | 10 | | Keine | (60' = 0 %) |
| I | | 2 | | 52' | |
| | 1 | | 20 | Keine | (60' = 0 %) |
| | 2 | | 20 | Keine | (60' = 0 %) |
| | 3 | | 20 | Keine | (60' = 2 %) |
| | 4 | | 20 | 43' | |
| | 5 | | 20 | Keine | (60' = 0 %) |
| | 6 | | 20 | 42' | |
| | 7 | | 20 | 34' | |

**Tabelle C - Fortsetzung**

**$KT_{50}$-Aerosoltest mit gegen Pyrethroide resistenten männlichen Musca domestica (Stamm Hans)**

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| 8 | 20 | Keine (60' = 20 %) |
| 9 | 20 | Keine (60' = 0 %) |
| 10 | 20 | Keine (60' = 0 %) |
| 11 | 20 | Keine (60' = 15 %) |
| 12 | 20 | Keine (60' = 0 %) |
| 13 | 20 | Keine (60' = 0 %) |
| 14 | 20 | Keine (60' = 0 %) |
| D + 11 | 10 + 10 | 41' |
| E + 11 | 10 + 10 | 44' |
| E + 11 | 10 + 5 | 42' |
| F + 11 | 4 + 20 | 14' |
| F + 11 | 4 + 10 | 10' |
| F + 11 | 4 + 5 | 19' |

**Tabelle C - Fortsetzung**

$KT_{50}$-Aerosoltest mit gegen Pyrethroide resistenten männlichen Musca domestica (Stamm Hans)

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| F + 11 | 4 + 2 | 21' |
| F + 11 | 4 + 1 | 30' |
| G + 11 | 4 + 10 | 40' |
| H + 11 | 10 + 20 | 40' |
| H + 11 | 10 + 10 | 53' |
| I + 1 | 2 + 2 | 25' |
| I + 2 | 2 + 2 | 43' |
| I + 3 | 2 + 2 | 45' |
| I + 4 | 2 + 2 | 47' |
| I + 8 | 2 + 2 | 17' |
| I + 9 | 2 + 2 | 38' |
| I + 10 | 2 + 2 | 40' |
| I + 11 | 2 + 10 | 32' |

**Tabelle C** - Fortsetzung

$KT_{50}$-Aerosoltest mit gegen Pyrethroide resistenten männlichen Musca domestica (Stamm Hans)

| Wirkstoffe / Synergisten | Konzentration in $mg/m^3$ Wirkstoffe + Synergisten | $KT_{50}$ innerhalb v. 60 Minuten (% K. D. nach 60 Minuten) |
|---|---|---|
| I + 11 | 2 + 8 | 32' |
| I + 11 | 2 + 4 | 34' |
| I + 11 | 2 + 2 | 48' |
| I + 11 | 2 + 1 | 36' |
| I + 12 | 2 + 2 | 36' |
| I + 13 | 2 + 2 | 33' |

**Ansprüche**

1. Halogenalkyl-, Alkenyl-und Alkinyl-azole der allgemeinen Formel (I)

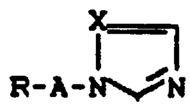

(I)

in welcher
X für ein Stickstoffatom oder eine CH-Gruppierung steht,
R für gegebenenfalls substituiertes tert.-Alkyl oder für gegebenenfalls substituiertes Cycloalkyl steht und
A für eine der Gruppierungen

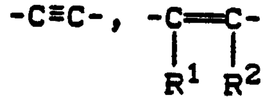

oder

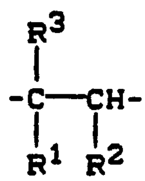

steht,
worin
$R^1$ für Halogen, Alkoxy oder Trialkylsilyloxy steht,
$R^2$ für Wasserstoff oder Halogen steht und
$R^3$ für Halogen steht,
sowie ihre Salze.

2. Verbindungen gemäß Anspruch 1, in welchen
X für ein Stickstoffatom oder eine CH-Gruppierung,
R für gegebenenfalls durch Halogen substituiertes tert.-Alkyl mit 4 bis 8 Kohlenstoffatomen oder für gegebenenfalls durch Alkyl mit 1 bis 4 Kohlenstoffatomen und/oder Halogen substituiertes Cycloalkyl mit 3 bis 6 Kohlenstoffatomen steht, und
A für die Gruppierungen

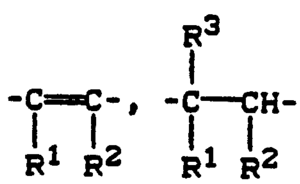

oder -C≡C-steht,
wobei
$R^1$ für Halogen, Alkoxy mit 1 bis 4 Kohlenstoffatomen oder für Trimethylsilyloxy steht,
$R^2$ für Wasserstoff oder Halogen steht und
$R^3$ für Halogen steht.

3. Verbindungen gemäß Anspruch 1, in welchen
X für ein Stickstoffatom oder eine CH-Gruppierung steht,
R für gegebenenfalls durch Chlor und/oder Fluor substituiertes tert.-Alkyl mit 4 bis 8 Kohlenstoffatomen

oder für gegebenenfalls durch Chlor, Methyl, Ethyl und/oder Isopropyl substituiertes Cyclopropyl, Cyclopentyl oder Cyclohexyl steht und
A für die Gruppierung -C≡C-steht oder
A für die Gruppierung

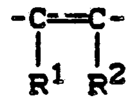

steht,
in welcher
(a) $R^1$ für Chlor, Methoxy, Ethoxy oder Trimethylsilyloxy steht und
$R^2$ für Wasserstoff steht, oder
(b) $R^1$ und $R^2$ für Chlor stehen, oder
A für die Gruppierung

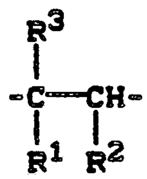

steht,
worin
$R^1$, $R^2$ und $R^3$ für Chlor stehen.

4. Verbindung der Formel

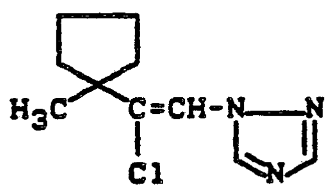

5. Verfahren zur Herstellung der Halogenalkyl-, Alkenyl-und Alkinyl-azole der allgemeinen Formel (I)

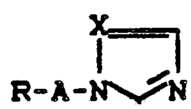 (I)

in welcher
X für ein Stickstoffatom oder eine CH-Gruppierung steht,
R für gegebenenfalls substituiertes tert.-Alkyl oder für gegebenenfalls substituiertes Cycloalkyl steht und
A für eine der Gruppierungen

-C≡C-, 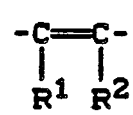

oder

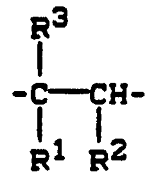

steht,
worin
$R^1$ für Halogen, Alkoxy oder Trialkylsilyloxy steht,
$R^2$ für Wasserstoff oder Halogen steht und
$R^3$ für Halogen steht,
sowie ihre Salze,
dadurch gekennzeichnet, daß man
(a) für den Fall, daß X und R die oben angegebenen Bedeutungen haben und
A für die Gruppierung

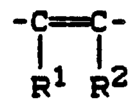

steht, worin
$R^1$ für Halogen steht und
$R^2$ für Wasserstoff steht,
Azolyl-Ketone der allgemeinen Formel (II)

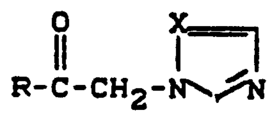
(II)

in welcher
X und R die oben angegebenen Bedeutungen haben,
in an sich bekannter Weise mit geeigneten Halogenierungsmitteln umsetzt, oder
(b) für den Fall, daß X und R die oben angegebenen Bedeutungen haben und
A für die Gruppierung

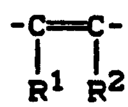

steht, worin
$R^1$ für Alkoxy oder Trialkylsilyloxy steht und
$R^2$ für Wasserstoff steht,
Azolyl-Ketone der allgemeinen Formel (II)

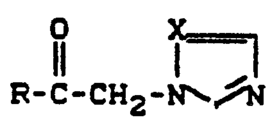
(II)

in welcher
X und R die oben angegebenen Bedeutungen haben,
in an sich bekannter Weise in Gegenwart einer starken Base und in Gegenwart eines Verdünnungsmittels mit einem Alkylierungsmittel bzw. einem Silylierungsmittel umsetzt, oder
(c) für den Fall, daß X und R die oben angegebenen Bedeutungen haben und
A für die Gruppierung $-C\equiv C-$steht,
Chlorvinyl-azol-Derivate der allgemeinen Formel (Ia)

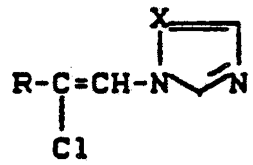

(Ia)

in welcher

X und R die oben angegebenen Bedeutungen haben,

durch Umsetzung mit einer starken Base in Gegenwart eines Verdünnungsmittels dehydrochloriert, oder

(d) für den Fall, daß X und R die oben angegebenen Bedeutungen haben und

A für die Gruppierung

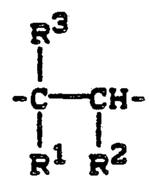

steht, worin

$R^1$, $R^2$ und $R^3$ die oben angegebene Bedeutung haben,

Alkenyl-azole der allgemeinen Formel (Ib)

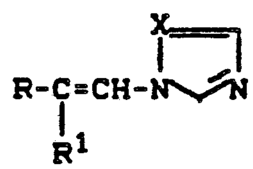

(Ib)

in welcher

X, R und $R^1$ die oben angegebenen Bedeutungen haben,

mit Halogenverbindungen der Formel (III)

$R^2$-$R^3$ (III)

in welcher

$R^2$ und $R^3$ die oben angegebene Bedeutung haben,

in Gegenwart eines Verdünnungsmittels umsetzt, oder

(e) für den Fall, daß X und R die oben angegebenen Bedeutungen haben und

A für die Gruppierung

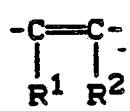

steht, worin

$R^1$ die oben angegebene Bedeutung hat und

$R^2$ für Halogen steht,

Halogenalkyl-azole der Formel (Ic)

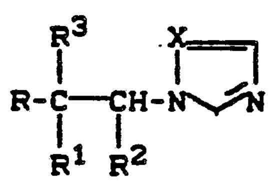

(Ic)

in welcher

X, R und $R^1$ die oben angegebenen Bedeutungen haben und

$R^2$ und $R^3$ für Halogen stehen,
durch Umsetzung mit einer starken Base in Gegenwart eines Verdünnungsmittels dehydrohalogeniert und gegebenenfalls an die so erhaltenen Verbindungen der Formel (I) eine Säure oder ein Metallsalz nach üblichen Methoden addiert.

6. Verwendung der Verbindungen der Formel (I) gemäß den Ansprüchen 1 oder 5 und ihrer Salze als Synergisten zur Schädlingsbekämpfung.

7. Mischungen aus Verbindungen der Formel (I) gemäß den Ansprüchen 1 oder 5 und ihrer Salze mit Photosynthesehemmer-Herbiziden oder mit Arthropodiziden.

8. Mischungen aus Verbindungen der Formel (I) gemäß den Ansprüchen 1 oder 5 und ihrer Salze mit arthropodiziden Wirkstoffen aus den Gruppen

1. Carbamidsäureester und/oder
2. Carbonsäureester, einschließlich der natürlichen und synthetischen Pyrethroide und/oder
3. Phosphorverbindungen, wie Phosphorsäure-und Phosphonsäureestern, einschließlich der Thiol-und Thionoverbindungen.

9. Schädlingsbekämpfungsmittel, welche wenigstens eine Mischung gemäß den Ansprüchen 7 oder 8 enthalten.

10. Verwendung der Mischungen gemäß den Ansprüchen 7 oder 8 sowie der Schädlingsbekämpfungsmittel gemäß Anspruch 9 zur Bekämpfung von Schädlingen.

11. Verfahren zur Herstellung von Schädlingsbekämpfungsmitteln, dadurch gekennzeichnet, daß man wenigstens eine Mischung gemäß den Ansprüchen 7 oder 8 mit Streckmitteln und/oder oberflächenaktiven Mitteln vermischt.

Europäisches Patentamt

# EUROPÄISCHER RECHERCHENBERICHT

Nummer der Anmeldung

EP 88 10 0037

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 089 920 (CIBA-GEIGY)<br>* Ansprüche *<br>--- | 1 | C 07 D 249/08<br>C 07 D 233/56<br>A 01 N 43/653<br>A 01 N 43/50<br>C 07 F 7/18 |
| Y | EP-A-0 160 931 (BAYER AG)<br>* Ansprüche *<br>--- | 1 | |
| A | EP-A-0 015 002 (THE WELLCOME FOUNDATION)<br>--- | | |
| P,X | DE-A-3 525 978 (BAYER AG)<br>* Insgesamt *<br>----- | 1-11 | |
| | | | **RECHERCHIERTE SACHGEBIETE (Int. Cl.4)** |
| | | | C 07 D 249/00<br>C 07 D 233/00<br>A 01 N 43/00<br>C 07 F 7/00 |

Der vorliegende Recherchenbericht wurde für alle Patentansprüche erstellt

| Recherchenort | Abschlußdatum der Recherche | Prüfer |
|---|---|---|
| DEN HAAG | 26-04-1988 | BRIGHENTI |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)